# EUROPEAN PATENT APPLICATION

(11) **EP 2 266 579 A1**
(43) Date of publication of application: **29.12.2010**
(21) Application number: 10177224.2
(22) Date of filing: 18.10.2005
(51) Int. Cl.: A61K 31/70, A61K 31/55, A61K 31/535, A61P 31/14, A61P 31/18, A61K 9/00

(54) **Pharmaceutical composition comprising elvucitavine**

(30) Priority: 19.10.2004 US 620023 P
(62) Divisional of application: 05813058.4
(71) Applicant: Achillion Pharmaceuticals, Inc., New Haven, CT 06511-6624 (US)
(72) Inventor: Pottage, John, Madison, CT 06443 (US)
(74) Representative: Smaggasgale, Gillian Helen

(57) **Abstract**

A pharmaceutical composition comprising elvucitabine, lactose monohydrate, and anhydrous lactose, in which the lactose monohydrate and anhydrous lactose taken together comprise about 60 percent to about 90 percent of the pharmaceutical composition by weight.

## Description

### CROSS REFERENCE TO RELATED APPLICATIONS

This application claims priority to US provisional patent application no. 60/620,023, filed October 19, 2004, which is hereby incorporated by reference in its entirety.

### FIELD OF THE INVENTION

A method of treating viral infections, particularly Hepatitis B (HBV) and Human Immunodeficiency Virus (HIV), by administering a low dose of Elvucitabine in combination with a second active agent to a patient suffering viral infection is provided herein. The Elvucitabine dosage may be given BID, daily, once every 48 hours, or once weekly. Also provided herein are packaged pharmaceutical compositions comprising Elvucitabine and a second active agent and instructions for treating a viral infection by administering a low BID, daily, once/ 48 hour, or weekly dosage of Elvucitabine. Methods of improving patient compliance with anti-viral therapy by providing a reverse transcriptase inhibitor formulated for low dose administration in combination with a second active agent are also included herein.

### BACKGROUND

Hepatitis B virus (HBV) infection is a major health problem throughout the world. HBV is a causative agent of both an acute and chronic form of hepatitis. It is estimated that more than 200 million people worldwide are chronic carriers of HBV.

HBV belongs to the family Hepadnaviridae, which includes a number of related viruses that primarily infect small rodents. All members of the hepadnavirus family have a number of characteristics in common such as morphological appearance, antigenic makeup and DNA size and structure. Pathological findings following infection with the members of this family are quite similar. Studies show that the replication and spread of the viruses of this family are dependent upon the reverse transcriptase of an RNA intermediate.

HBV is a double-stranded DNA virus. Its DNA polymerase catalyzes both DNA-dependent and RNA-dependent RNA synthesis. The life cycle of HBV involves the enzyme reverse transcriptase in its DNA replication.

Although acute HBV infections are generally self-limiting, in many instances the disease can progress to the chronic state. HBV infection also creates a risk to fulminant hepatitis. In addition, Hepatitis B viral infections are closely associated with hepatocellular carcinoma.

AIDS is a generally fatal disease caused by a human pathogenic retrovirus known as human immunodeficiency virus (HIV), which includes HIV-1 and HIV-2. Reverse transcriptase plays an essential role in the elaboration and life cycle of HIV and consequently, the progress of the disease. Reverse transcriptase inhibitors are currently used with other classes of anti-viral agents to slow and in some cases halt the progress of HIV infection.

Reverese transcriptase inhibitors are preferred therapeutics for treating certain viral infections, particularly HBV and HIV infections. Typically about 300 mg of a reverse transciptase inhibitor must be administered daily for effective treatment of a viral infections, sometimes on a once per day dosing schedule, but more typically on a twice or three times per day dosing schedule. Because patients suffering from HBV or HIV often take a number of medications, a reverse transcriptase inhibitor efficacious at lower dosages is urgently needed. A reverse transcriptase inhibitor that can be administered once daily or less frequently is particularly desireable.

Elvucitabine is a nucleoside analog of the formula

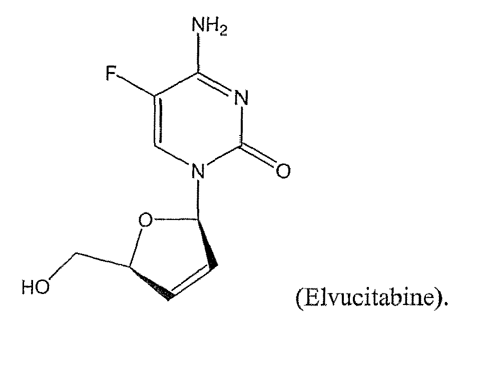

The anti-viral properties of Elvucitabine have been described previously in US patent nos. 5,621,120, 5,627,160, and 5,839,881, and US patent application no. 10/411,929, filed April 11, 2004, which are hereby incorporated by reference for their teachings regarding the use of Elvucitabine for treating viral infections, including HBV and HIV infections, and for teachings regarding the chemical synthesis of Elvucitabine.

Agents useful for treating viral infections, including HBV and HIV infections, are often most effective when combined with additional anti-viral or immunomodulatory agents. New combinations of anti-viral agents or anti-viral and immunomodulatory agents are therefore desirable. Such combinations efficacious when administered at low dosages are particularly desirable. The present invention fulfills this need, and provides further related advantages.

### SUMMARY OF THE INVENTION

A pharmaceutical composition comprising Elvucitabine and a second active agent, wherein the second active agent is an immunomodulatory compound or an antiviral agent or a combination comprising one or more of the foregoing active agents, is provided herein. In certain embodiments second active agent used in combination with Elvucitabine is not interferon, a nucleoside or a nucleoside analog.

It has been discovered that Elvucitabine, a reverse transcriptase inhibitor, is efficacious for treating viral infections, including HIV and HBV infections, when administered at very low dosages. Elvucitabine is an effective anti-viral agent (including anti-HIV efficacy) when as little as about 2.5 mg to about 10 mg is administered once a day. For some patients, including patients having an HIV or HBV infection, Elvucitabine may be effective when administered as infrequently as one time per week. Thus pharmaceutical compositions comprising Elvucitabine and a second active agent, wherein the second active agent is an immunomodulatory compound or an antiviral agent or a combination comprising one or more of the foregoing active agents are provided herein. Dosage forms comprising at least 2.5 mg Elvucitabine, but less than 70 mg and preferably less than 7 mg Elvucitabine, and a second active agent are provided herein.

Methods of treating viral infections, including HIV and HBV infections, comprising administering about 2.5 mg to about 10 mg Elvucitabine per day, or about 5 mg to about 20 mg Elvucitabine per 48 hour interval, or about 40 mg to about 100 mg Elvucitabine per week in combination with a second active agent, such as an immunomodulatory compound or an antiviral agent, to a patient having a viral infecition, such as an HIV or HBV infection are also provided herein. Methods of administering the Elvucitabine as an oral dosage form are preferred. An embodiment comprising treating viral infection in a patient in need thereof by administering to the patient from about 2.5 mg to not more than 6.5 mg Elvucitabine per day in combination with a second active agent is included herein.

Methods of treating viral infections in a patient in need thereof, such as HBV and HIV infections, comprising administering Elvucitabine in combination with a second active agent to the patient, are also provided herein. The second active agent may comprise a combination of two or more active agents. Methods of treatment in which Elvucitabine is administered in the same dosage form as the second active agent, as well as methods in which Elvucitabine and the second active agent are administered in separate dosage forms, but packaged together, and methods in which Elvucitabine and the second active agent are prescribed together are included herein.

Pharmaceutical compositions suitable for once per day administration comprising about 2.5 mg to about 10 mg Elvucitabine, once per 48 hour interval administration comprising about 5 mg to about 20 mg Elvucitabine per 48 hour interval, or once per week administration comprising about 40 mg to about 100 mg Elvucitabine, wherein the Elvucitabine is combined with second active agent, such as an immunomodulatory compound or an antiviral agent, are provided herein.

Packaged pharmaceutical compositions comprising an Elvucitabine pharmaceutical composition in a container and instructions for using the composition for treating a viral infection by administering Elvucitabine in combination with a second active agent to a patient in need thereof, wherein in the second active agent is an immunomodulatory agent or an anti-viral agent are provided herein. In certain embodiments second active agent used in combination with Elvucitabine is not interferon, a nucleoside, or a nucleoside analog.

In the pharmaceutical compositions, dosage forms, including oral dosage forms, and methods of treatment described herein the second active agent may be an anti-viral agent such as a tyrosine kinase inhibitor, a CCR5 inhibitor, a non-nucleoside reverse transcriptase inhibitor, a protease inhibitor, or an integrase inhibitor. The second active agent may also be a combination comprising one or more of the foregoing active agents. Certain embodiments provided herein include pharmaceutical preparations containing Elvucitabine and two other active agents. For example embodiments including Elvucitabine, another nucleoside analog, and either a non-nucleoside reverse transcriptase inhibitor, such as efavirenz (SUSTIVA) or a protease inhibitor are included herein.

### BRIEF DESCRIPTION OF DRAWINGS

Figure 1 illustrates a PK model of Elvucitabine.

Figure 2 illustrates an example of individual fit using Pop PK analysis (IT2S).

Figure 3 illustrates the change in mean white blood cell count from baseline for 5 mg, 20mg, and 50mg Elvucitabine PD study over 20 weeks.

Figure 4 illustrates the change in mean absolute neutrophils from baseline for 5 mg, 20mg, and 50mg Elvucitabine PD study over 20 weeks.

Figure 5 illustrates simulated plasma concentrations and mean observed ANC for 5 mg QD dose.

Figure 6 illustrates simulated plasma concentrations and mean observed ANC for 20 mg QD dose.

Figure 7 illustrates simulated plasma concentrations and mean observed ANC for 50 mg QD dose.

Figure 8 is a schematic of the relationship ofPK versus PD for Elvucitabine.

Figure 9 is an example of a simulated dosing regimen of a 10 mg QD.

Figure 10 is an example of a simulated dosing regimen of a 40 mg Q1week.

### DETAILED DESCRIPTION OF THE INVENTION

### TERMINOLOGY

Prior to setting forth the invention in detail, it may be helpful to provide definitions of certain terms to be used herein. Compounds of the present invention are described using standard nomenclature. Unless defined otherwise, all technical and scientific terms used herein have the same meaning as is commonly understood by one of skill in the art to which this invention belongs.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including", and "containing" are to be construed as open-ended terms (i.e., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. "About" indicates an approximate amount, including the quantity it modifies. When "about" is used to modify a quantity of Elvucitabine, the free, unsalted form is the type of form of Elvucitabine referred to, unless another Elvucitabine form is expressly stated. No language in the specification should be construed as indicating any non-claimed element as essential to the practice of the invention.

The term "Elvucitabine" is meant to include solvates (including hydrates) of the free compound or salt, crystalline and non-crystalline forms, isotopically enriched or labeled forms, as well as various polymorphs of Elvucitabine, i.e. 4-amino-5-fluoro-1-((2R,5S)-5-(hydroxymethyl)-2,5-dihydrofuran-2-yl)pyrimidin-2(1H)-one.

Elvucitabine contains asymmetric elements and can exist in different stereoisomeric forms. Elvicitabine can be, for example, a racemate or optically active form. While the 4-amino-5-fluoro-1-((2R,5S)-5-(hydroxymethyl)-2,5-dihydrofuran-2-yl)pyrimidin-2(1H)-one is preferred, methods of using racemic mixtures ofElvucitabine, and other optically pure stereoisomers of this compounds are within the scope of this invention.

Isotopes include those atoms having the same atomic number but different mass numbers. By way of general example, and without limitation, isotopes of hydrogen include tritium and deuterium and isotopes of carbon include 11C, 13C, and 14C.

An "active agent" is any compound, element, or mixture, that when administered to a patient alone or in combination with one or more other agents confers a therapeutic benefit on the patient. When the active agent is a compound solvates (including hydrates) of the free compound or salt, crystalline and non-crystalline forms, as well as various polymorphs or the compound are included. For example, an active agent can include optical isomers of the compound and pharmaceutically acceptable salts thereof either alone or in combination.

In some embodiments the second active agent is a "low dose active agent." Within the art of HIV treatment certain compounds that enhance the activity of antiviral agents by inhibiting liver enzymes that breakdown antiviral agents are sometimes referred to as "low dose active agents". Ritonovir (NORVIR, Abbot Laboratories, Abbott Park, IL) is an example of such a compound. While low dosages of Elvucitabine may be administered with ritonovir, the term "low dose active agent" is used herein without refererring to a particular mechanism of action to describe any compound that retains therapeutic efficacy when administered in small amounts, typically about 2.5 mg to about 10 mg daily, about 5 to about 20 mg every 48 hours, or about 40 to about 100 mg once weekly, or on another infrequent dosage schedule, typically once daily or less frequently.

"BID administration" is twice daily administration of a compound. Typically, each day two equal dosages of a therapeutic compound are given during waking hours. "QD administration" is once daily administration. "TID administration" is administration of a therapeutic compound three times daily. Typically three equal dosages are given each day during waking hours.. Some compounds administered BID or TID equal dosages are given with food, several hours apart, during waking hours.

The term "dosage form" denotes a form of a pharmaceutical composition that contains an amount sufficient to achieve a therapeutic effect with a single administration. The term "oral dosage form" is meant to include a unit dosage form prescribed or intended for oral administration. An oral dosage form may or may not comprise a plurality of subunits such as, for example, microcapsules or microtablets, packaged for administration in a single dose.

The term "effective amount" means an amount effective, when administered to a human or non-human patient, to provide any therapeutic benefit such as an amelioration of symptoms, e.g., an amount effective to decrease the symptoms of a viral infection, and preferably an amount sufficient to reduce the symptoms of an HBV or HIV infection. In certain circumstances a patient suffering from a viral infection may not present symptoms of being infected. Thus a therapeutically effective amount of a compound is also an amount sufficient to provide a positive effect on any indicia of disease, e.g. an amount sufficient to prevent a significant increase or significantly reduce the detectable level of virus or viral antibodies in the patient's blood, scrum, or tissues. A significant increase or reduction in the detectable level of virus or viral antibodies is any detectable change that is statistically significant in a standard parametric test of statistical significance such as Student's T-test, where p < 0.05.

A "nucleoside" is a molecule that includes a purine or pyrimidine base covalently bound to a ribose sugar. A "nucleoside analog" is synthetic molecule that resembles a naturally occurring nucleoside, but that lacks a bond site needed to link it to an adjacent nucleotide. For example a nucleoside analog may include a purine or pyrimidine base covalently bound to a hydroxyl substituted alkoxy group rather that a ribose sugar. Nucleoside analogs include AZT, acyclovir, combivir, abacavir, emtricitabine, ddI, ddC, d4T, L-FMAU (stavudine),and 3TC. Nucleotide analogs, which include a phosphate group, are not included within the definition of nucleoside analogs.

A "patient" is any human or non-human animal in need of medical treatment. Medical treatment can include treatment of an existing condition, such as a disease or disorder, or prophylactic or preventative treatment. In some embodiments the patient is a human patient.

"Pharmaceutically acceptable salts" includes derivatives of the disclosed compounds, wherein the parent compound is modified by making non-toxic acid or base addition salts thereof, and further refers to pharmaceutically acceptable solvates, including hydrates, of such compounds and such salts. Examples of pharmaceutically acceptable salts include, but are not limited to, mineral or organic acid addition salts of basic residues such as amines; alkali or organic addition salts of acidic residues such as carboxylic acids; and the like, and combinations comprising one or more of the foregoing salts. The pharmaceutically acceptable salts include non-toxic salts and the quaternary ammonium salts of the parent compound formed, for example, from non-toxic inorganic or organic acids. For example, non-toxic acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, and combinations comprising one or more of the foregoing salts.

Pharmaceutically acceptable organic salts include salts prepared from organic acids such as acetic, trifluoroacetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH2)n-COOH where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparginate, glutamate, and the like, and combinations comprising one or more of the foregoing salts.

"Pharmacokinetic parameters," (PK) are parameters, which describe the in vivo characteristics of Elvucitabine over time, including for example the *in vivo* dissolution characteristics and plasma concentration of Elvucitabine or other active agent. By "C*ₘₐₓ*" is meant the measured concentration of Elvucitabine in the plasma at the highest observed concentration. By *"*C*₂₄*" is meant the concentration of Elvucitabine in the plasma at about 24 hours. The term "T*ₘₐₓ*" refers to the time at which the concentration of Elvucitabine in the plasma is the highest. "AUC" is the area under the curve of a graph of the concentration of Elvucitabine (typically plasma concentration) vs. time, measured from one time to another.

By "subunit" is meant to include a composition, mixture, particle, etc., that can provide an oral dosage form alone or when combined with other subunits. By "part of the same subunit" is meant to refer to a subunit comprising certain ingredients. For example, a subunit comprising Elvucitabine or other active agent and an additional active ingredient may be placed together with additional subunits in a capsule to provide an oral dosage form.

### PHARMACOKINETICS AND PHARMACODYNAMICS MODELING TO OPTIMIZE THE THERAPEUTIC INDEX OF ELVLTCITABINE

Pharmacokinetics and pharmacodynamics modeling was used to explore potential dosing regimens for Elvucitabine. A pharmacokinetics (PK) model was developed to describe both the plasma concentration-time data and excreted urinary amounts from previously completed studies with Elvucitabine. With the PK model in hand and pharmacodynamics (PD) results of previously completed studies, the relationship of PK and PD from an efficacy and toxicity standpoint is obtained. From the results of the PK and PD studies, a therapeutic window of Elvucitabine is defined and simulated dosing regimens ranging from 5 mg BID to 100 mg Q1 week were explored using ADAPT II® Pharmacokinetic and Pharmacodynamic Systems Analysis Software. From the simulated dosing regimen results, ten dosing regimens were identified allowing maintenance therapy with Elvucitabine at levels effective against both HTV and HBV while avoiding bone toxicity.

### Pharmacokinetics:

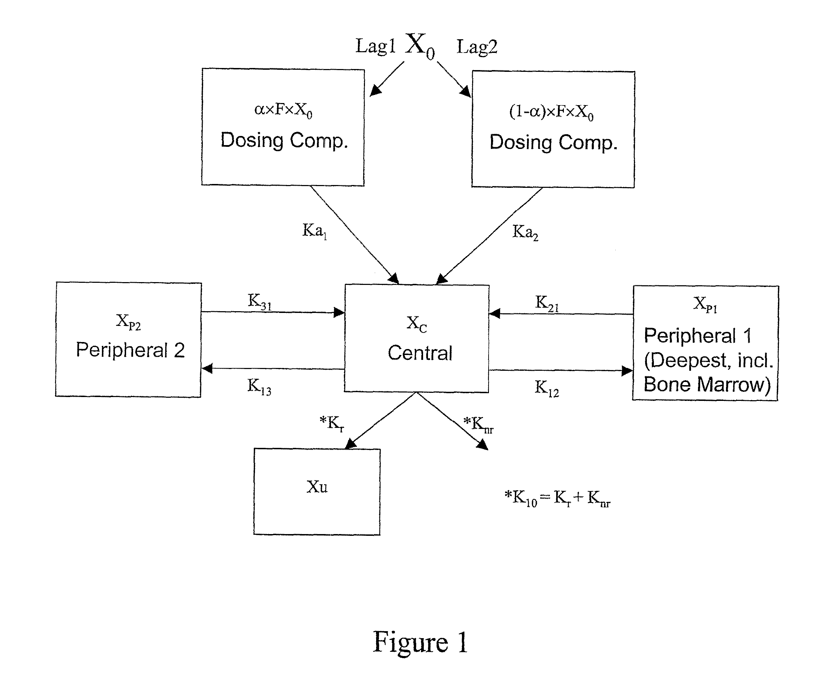

The PK model created included an individual compartmental PK analysis using ADAPT-II® and Population PK analysis using an iterative two-stage population modeling technique (IT2S®) of plasma and urine data from a study of healthy subjects, n = 29, 1 x 20 mg tablet PO (ACH443-011). Figure 1 illustrates the PK model of Elvucitabine according to a three compartment model characterized by two absorption peaks, each associated with a lag (Tlag1 and Tlag2) time and a first order absorption rate constant (Ka1 and Ka2).

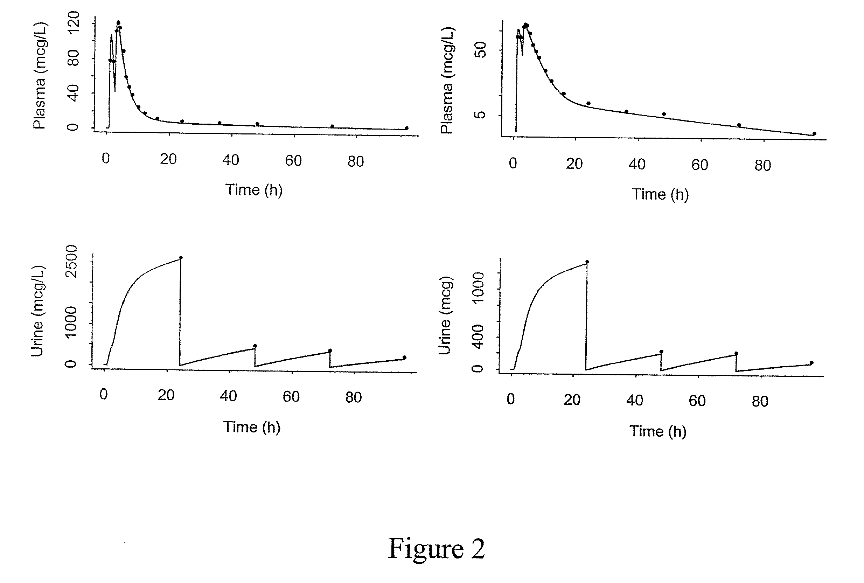

The Population PK parameters of Elvucitabine include a large apparent volume of distribution (Vss) of 1600 L; an early distribution (λ1) half-life of 0.59 hour, followed by another distribution (λ2) half-life of 30 hours; and an elimination (λz) half-life of 175 hours. Additional parameters include an oral clearance (CL/F) of 16 L/h and the fraction of drug excreted unchanged in urine (about 40%). Table 1 provides the Population PK parameters of Elvucitabine. Figure 2 illustrates an example of individual fit using a Population PK analysis (IT2S).

| Table 1. Population PK Parameters of Elvucitabine: | | | |
|---|---|---|---|
| Alpha | 0.338 | | |
| Tlag1 | 1.46 h | Ka1 | 1.1 1/h |
| Tlag2 | 2.76 h | Ka2 | 0.292 1/h |
| Vss/F | 1632 L | Vc/F | 30.6 L |
| | | Vp1/F | 1232 L |
| | | Vp2/F | 369 L |
| CL/F | 15.8 LJh | CLnr/F | 9.51 L/h |
| Fu | 39.6% | CLr | 6.29 L/h |
| T_{half1} | 0.586 h | K10 | 0.515 1/h |
| T_{half2} | 30.5 h | K12 | 0.246 1/h |
| T_{half3} | 175 h | K13 | 0.409 z1/h |
| | | K21 | 0.006 1/h |
| | | K31 | 0.034 1/h |

### Pharmacodynamics:

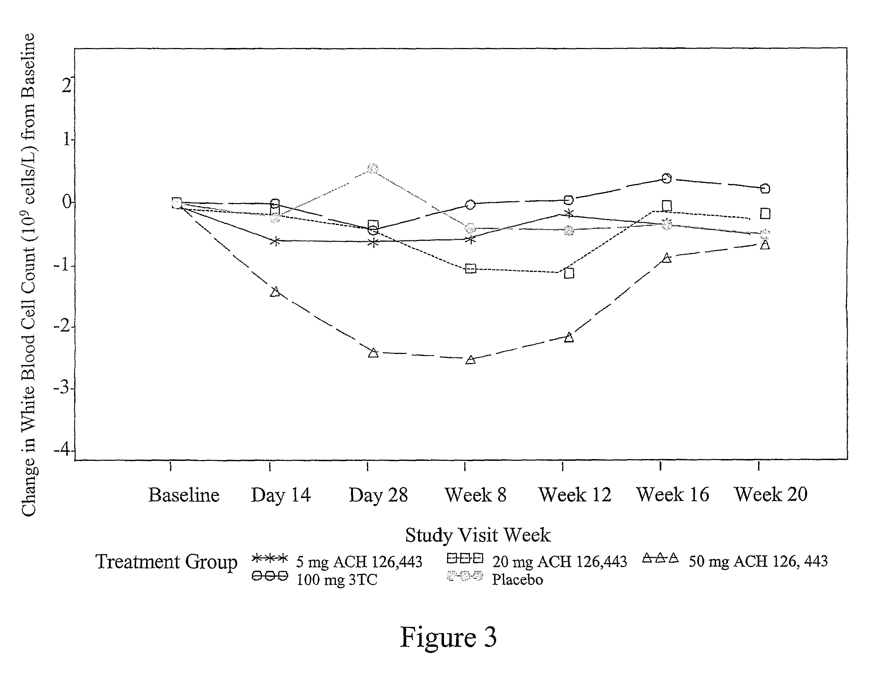

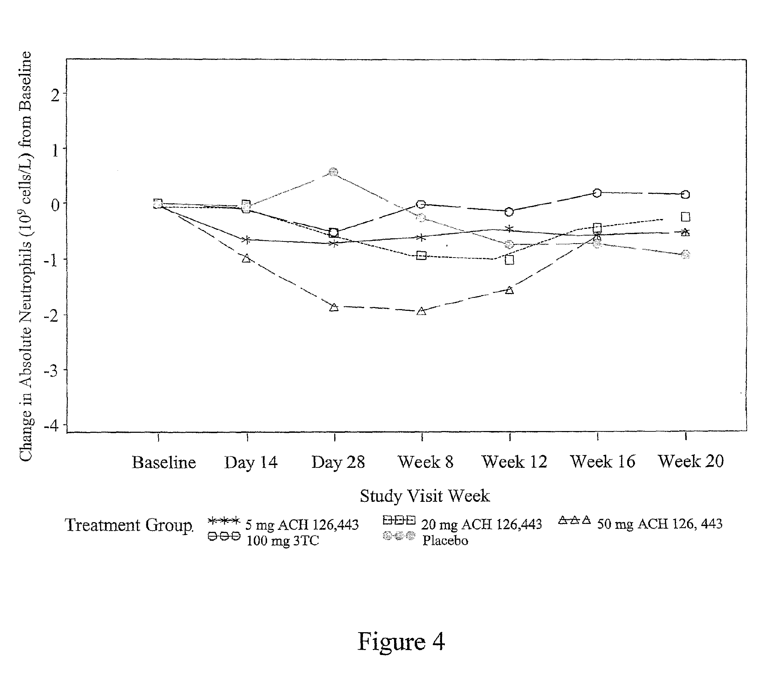

Pharmacodynamics of Elvucitabine was explored by analysis of hematology (absolute neutrophil count, ANC) data obtained from a 12 week study in HBV patients. Figure 3 illustrates the change in mean white blood cell count from baseline for three dosages of Elvucitabine (5 mg, 20 mg, and 50 mg), 100 mg 3TC, and placebo. Only the highest dose of Elvucitabine (50 mg) showed a significant decrease in white blood cell count. Figure 4 is a graphic representation of the change in absolute neutrophils from baseline for the same study. Table 2 provides absolute neutrophil count at 10⁹/L for the three doses resulting in a baseline estimated as the mean ANC of about 4x10⁹/L. A 25 percent decrease in the mean ANC corresponds to about 3x10⁹/L, the threshold for the start of toxicity.

**Table 2. PD data: Absolute Neutrophil Count (10⁹/L) (ANC)**

| Study Visit Week | | | | | |
|---|---|---|---|---|---|
| Study Treatment | Week 0 | Week 2 | Week 4 | Week 8 | Week 12 |
| 5 mg PO daily (n=17) | | | | | |
| Mean | 4.1 | 3.4 | 3.4 | 3.5 | 3.6 |
| (Range) | (1.6-6.9) | (1.5-6.8) | (1.4-6.4) | (1.1-5.9) | (1.7-5.2) |
| 20 mg PO daily (n=20) | | | | | |
| Mean | 3.8 | 3.7 | 3.2 | 2.8 | 2.7 |
| (Range) | (1.8-6.2) | (2.1-9.0) | (1.0-6.8) | (1.1-4.8) | (1.2-4.8) |
| 50 mg PO daily (n=19) | | | | | |
| Mean | 4.1 | 3.1 | 2.3 | 2.2 | 2.6 |
| (Range) | (1.7-7.7) | (1.5-8.2) | (0.9-5.6) | (0.8-5.7) | (1.0-5.7) |
| Placebo (n=8) | | | | | |
| Mean | 4.3 | 4.3 | 4.9 | 4.1 | 3.6 |
| (Range) | (1.8-8.4) | (1.7-9.6) | (1.8-10.7) | (2.1-8.8) | (2.4-6.4) |

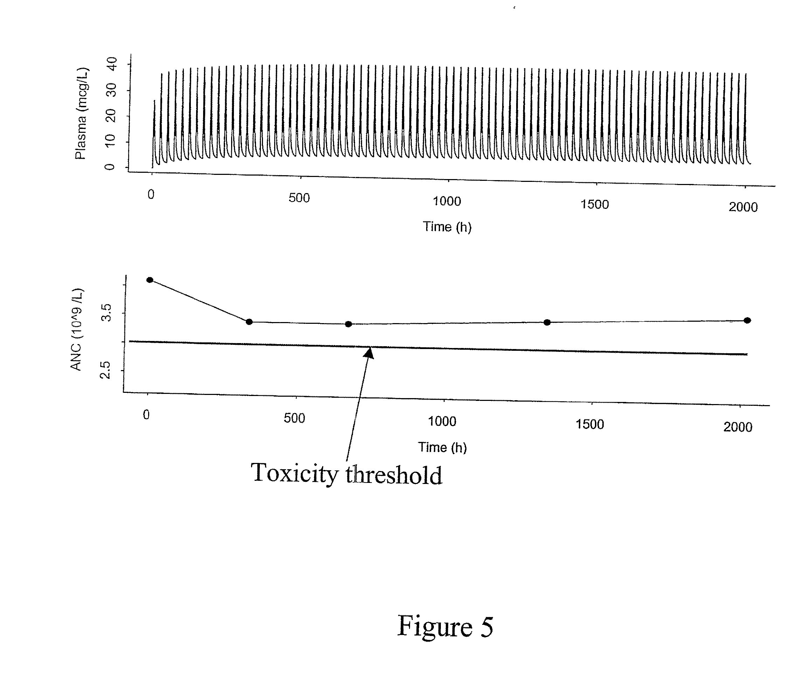

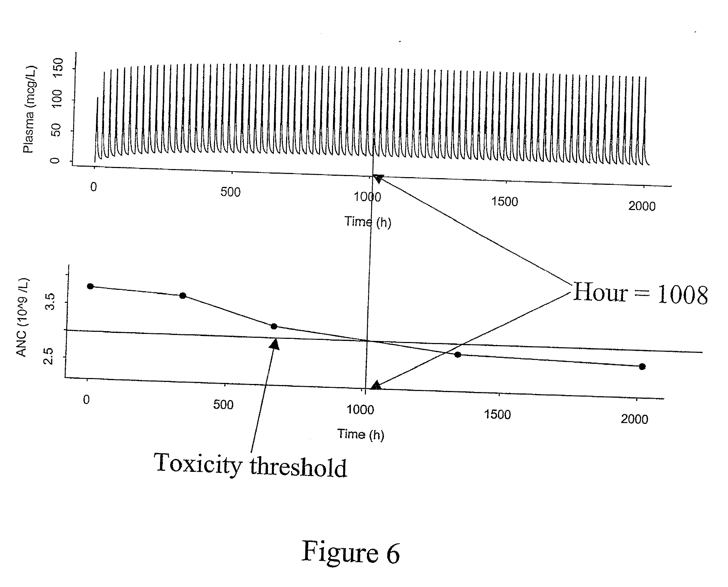

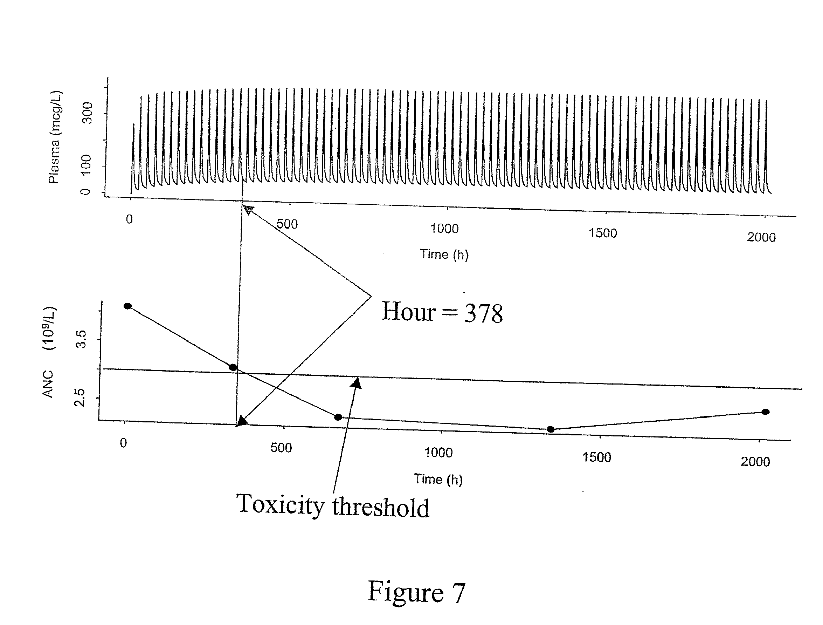

Figure 5 provides a plot of the mean observed ANC and a simulated concentration-time profile for the 5 mg QD dose. As shown by the graph, the toxicity threshold is not met by the dosing regimen. As provided in Figure 6, the 20 mg QD dose crosses the toxicity threshold at about 1008 hours. Increasing the dose to 50 mg QD results in reaching the toxicity threshold at 378 hours (Figure 7).

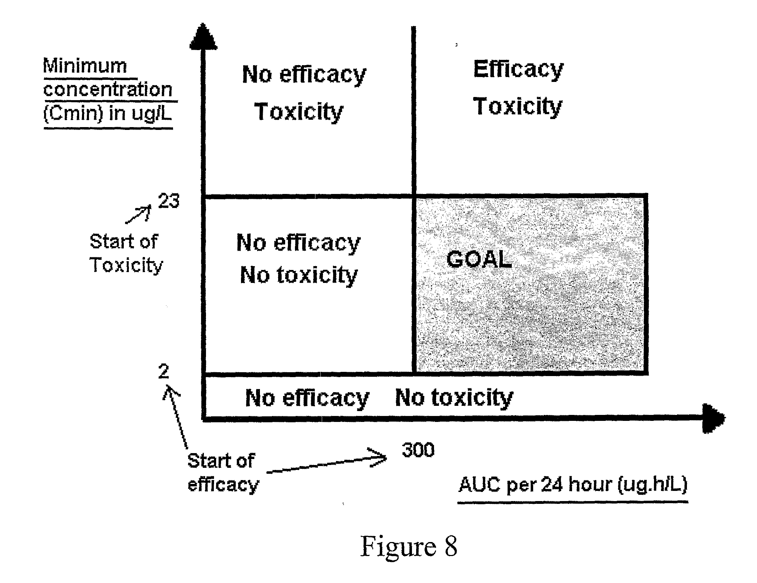

From the foregoing results, a therapeutic window of Elvucitabine was identified. It has been determined that a 5 mg QD dose is effective for HBV and, therefore, a plasma AUCss/24 of 300 µg·h/L was identified as the efficacy threshold. Furthermore, as Elvucitabine exhibits an *in vitro* IC₅₀ for HIV of 4.8 nM, which corresponds to 1.08 µg/L, the Cₘᵢₙ for efficacy was determined to be ≥2 µg/L. The onset of toxicity (ANC < 3x10⁹/L) linked to the deepest (P1) compartment is Cₘₐₓ > 40 µg/L, which correlates to a central compartment (plasma) Cₘᵢₙ ≥23 µg/L. A conservative therapeutic window is determined to have an AUCss/24 > 300 µg·h/L; Cₘᵢₙ Central ≥2 µg/L, but < 23 µg/L; and a Cₘₐₓ P1 < 40 µg/L. Figure 8 provides a schematic of the relationship of PK versus PD where the targeted Cₘᵢₙ and AUC/24 that provides efficacy without toxicity is desired.

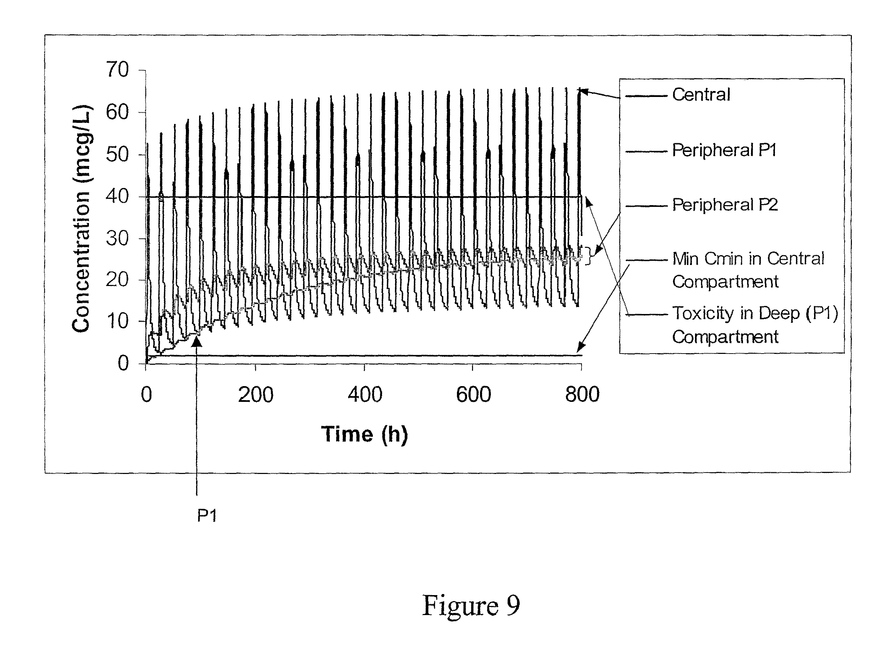

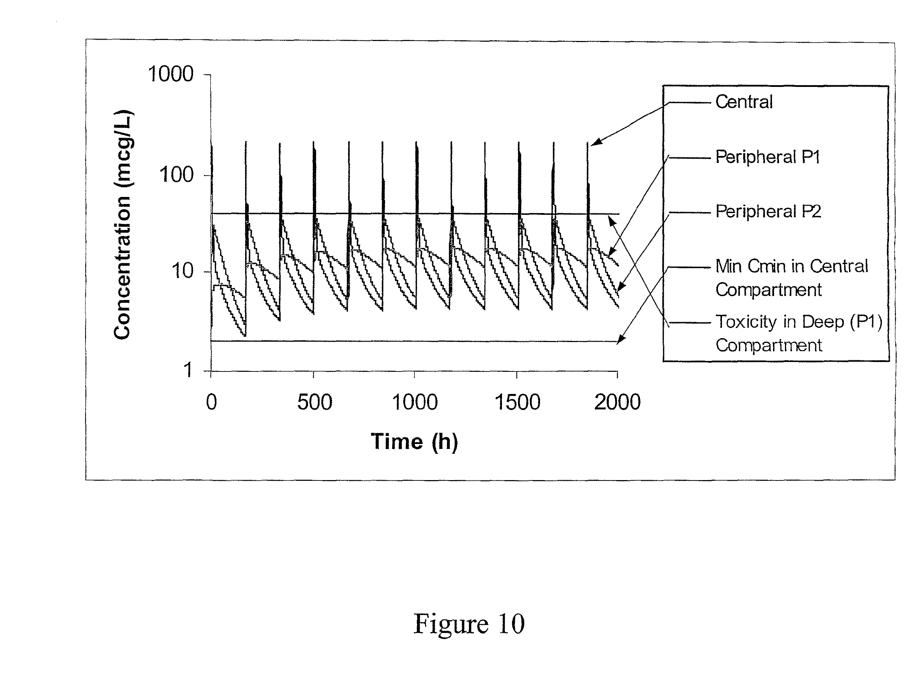

From the PK and PD analyses, twenty-nine dosing regimens were simulated using ADAPT II®. The dosing regimens simulated ranged from 5 mg twice a day to 100 mg Q1week and are provided in Table 3. An asterisk (*) indicates those results outside of the defined therapeutic window. From the study, ten dosing regimens were identified that meet the therapeutic window: one twice a day regimen at 5 mg; three once a day regimens at 5 mg, 7.5 mg, and 10 mg; three once every 48 hour regimens at 10 mg, 15 mg, and 20 mg; and three once a week regimens at 30 mg, 40 mg, and 50 mg. Surprisingly, three regimens with a once weekly dosing have been identified that would allow maintenance therapy with Elvucitabine at levels effective against both HIV and HBV while avoiding bone marrow toxicity. Figure 9 provides a simulated concentration-time profile of 10 mg QD dosing regimen while Figure 10 provides a profile for 40 mg Qlweek dosing. The 40 mg Qlweek dosing regimen, according to the model, provides the desired efficacy while at the same time avoiding the toxicity threshold.

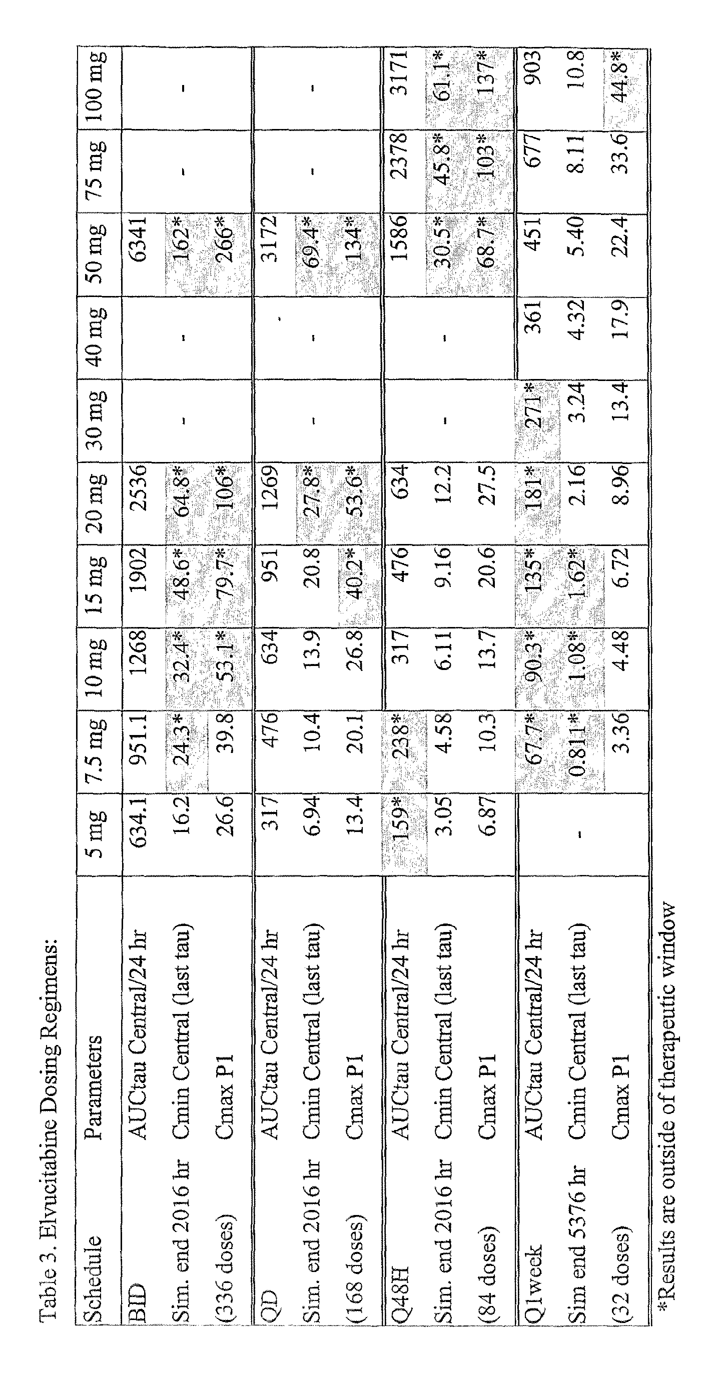

### ELVUCITABINE PHARMACEUTICAL COMBINATIONS

The Population PK parameters of Elvucitabine include a large apparent volume of distribution (Vss) of 1600 L; an early distribution (λ1) half-life of 0.59 hour, followed by another distribution (λ2) half-life of 30 hours; and an elimination (λz) half-life of 175 hours. Additional parameters include an oral clearance (CL/F) of 16 L/h and the fraction of drug excreted unchanged in urine (about 40%). Table 1 provides the Population PK parameters of Elvucitabine. Figure 2 illustrates an example of individual fit using a Population PK analysis (IT2S).Provided herein are combinations, for pharmaceutical use, comprising Elvucitabine and a second active agent. Pharmaceutical combinations may, for example, be in the form of a combination pharmaceutical composition or combination dosage form containing Elvucitabine and a second active agent or may be any combination in which Elvucitabine and a second active agent are administered together. The second active agent may contain a single active chemical entity or more than one active chemical entity. The second active agent may be an immunomodulatory compound, or an antiviral agent. In some embodiments the pharmaceutical composition comprises Elvucitabine and a second active agent, wherein the second active agent is an immunomodulatory compound or an antiviral agent or a combination comprising one or more of the foregoing active agents, with the proviso that the second active agent is not interferon, a nucleoside or a nucleoside analog. For example, the anti-viral agent may be a tyrosine kinase inhibitor, a CCR5 inhibitor, a non-nucleoside reverse transcriptase inhibitor, a nucleoside reverse transcriptase inhibitor, a protease inhibitor, a fusion inhibitor, an integrase inhibitor, or an immunomodulatory compound. The second active agent may be, or a combination comprising one or more of the foregoing active agents. In one embodiment, the second active agent is one that may be administered once per day or even less frequently. In another embodiment, the second active agent is a low dose active agent. In another embodiment the second active agent is not D4T (ZERIT®, Bristol-Myers Squibb, also known as stauvudine), carbovir, acyclovir (ZOVIRAX®, GlaxoSmithKline), 3TC, FTC (emtricitabine), RACIVIR® (a mixture of emtricitabine and its positive enantiomer), interferon, ddI (VIDEX®), ddC (Zalcitabine), or L-(-)-FMAU, Famciclovir, or Penciclovir or if Elvucitabine is combined with these active agents an effective amount, but not more than 6.5 mg of Elvucitabine is administered daily.

The second active agent may be a single chemical entity or may be comprised of more than one chemical entity. When the second active agent includes more than one active chemical entity Elvucitabine may be prepared in a single dosage form with one or more of the active chemical entities and the other(s) may be administered separately. Alternatively all active chemical entities may be combined with Elvucitabine in a single dosage form.

Combination combinations containing Elvucitabine and two or more other active agents are included herein. Certain embodiments pertain to combination containing Elvucitabine, an additional nucleoside analog, and either a non-nucleoside reverse transcriptase inhibitor or a protease inhibitor. The non-nucleoside reverse transcriptase inhibitor may be, for example, efavirenz (SUSTIVA, Bristol-Myers Squibb, New York, NY) or nevirapine (VIRAMUNE, Boehringer Ingelheim, Danbury, CT).

Immunomodulatory compounds, in the context of HBV and HIV treatment, include glucocorticoids, thalidomide, alpha interferon, and its analogs, IL-2, and hematopoietins.

The second active agent may be a single chemical entity or may be comprised of more than one chemical entity. When the second active agent includes more than one active chemical entity Elvucitabine may be prepared in a single dosage form with one or more of the active chemical entities and the other(s) may be administered separately. Alternatively all active chemical entities may be combined with Elvucitabine in a single dosage form.Glucocorticoids include, but are not limited to, hydrocortisone, cortisone, prednisone, prednisolone, methylprednisolone, triamcinolone, paramethasone, betamethasone, and dexamethasone.

Thalidomide analogs include, but are not limited to ACTIMID and REVIMID (Celgene, Warren, NJ).

Hematopoietins include, but are not limited to hematopoietin-1 and hematopoietin-2, and also include other members of the hematopoietin superfamily such as the various colony stimulating factors (e.g. (e.g. G-CSF, GM-CSF, M-CSF), Epo, SCF (stem cell factor), various Interleukins (IL-1, IL-3, IL-4, IL-5, IL-6, IL-11, IL-12), LIF, TGF-beta, TNF-alpha) and other low molecular weight factors (e.g. AcSDKP, pEEDCK, thymic hormone, and minicytokines).

The second active agent may comprise a tyrosine kinase inhibitor. Tyrosine kinases are a class of enzymes that catalyze the transfer of the terminal phosphate of adenosine triphosphate to the phenolic hydroxyl group of a tyrosine residue present in a target protein. Tyrosine kinases play a critical role in signal transduction for several cellular functions including cell proliferation, carcinogenesis, apoptosis, and cell differentiation. Tyrosine kinase inhibitors have been found to have antiviral properties and may be used as the second active agent. Tyrosine kinases inhibitors include Imatinib mesylate (GLEEVEC® or GLIVEC®, Novartis), Gefitinib (IRESSA®, Astra Zeneca), and erlotinib (TARCEVA, OSI Pharmaceuticals). Several other tyrosine kinase inhibitors are in phase II or phase III clinical trials. For example, AMN107 (Novartis Pharma AG) is slated to enter Phase II clinical trials for the treatment of chronic myeloid leukemia and sunitinib malate (also known as SU11248, brand name SUTENT®) (Pfizer) is in Phase II clinical trials for the treatment of cancer. Combinations of Elvucitabine and sunitinib malate and optionally AMN107 are within the scope of the invention.

The second active agent may comprise a CCR5 inhibitor. CCR5 is a receptor involved in HIV-1 fusion and entry. Tyrosines and negatively charged residues in the amino-terminal domain of CCR5 may be important for this function. Inhibitors of CCR5 include small molecules, peptides, chemokines and their derivatives, and monoclonal antibodies. CCR5 inhibitors currently being tested include GlaxoSmithKlines's GSK-873,140 (aplaviroc) and Schering Plough Corporation's SCH-417690 (vicriviroc). Other CCR5 inhibitors include 1-[(2,4-dimethyl-3-pyridinyl)carbonyl]-4-methyl-4-[3(S)- methyl-4-[1 (S)-[4-(trifluoromethyl)phenyl]ethyl]-1-piperazinyl]- piperidine N1-oxide; SCH-C; SCH-D; TAK-779; UK-427,857 or marviroc (Pfizer), antibodies to CCR5; and combinations comprising one or more of the foregoing CCR5 inhibitors.

The second active agent may comprise an additional nucleoside reverse transcriptase inhibitor. Reverse transcriptase inhibitors interfere with the reverse transcriptase enzyme and prevent the virus from replicating. Suitable nucleoside reverse transcriptase inhibitors include lamivudine (EPIVIR®, 3TC®, GlaxoSmithKline), zidovudine (RETROVIR® (AZT), GlaxoSmithKline), lamivudine and zidovudine combination (COMBIVIR®, GlaxoSmithKline), emtricitabine (EMTRIVA®, Gilead), abacavir and lamivudine (EPZICOM®, GlaxoSmithKline), zalcitabine (HIVED®, Roche US Pharmaceuticals), abacavir, zidovudine, and lamivudine (TRIZIVIR®, GlaxoSmithKline), tenofovir disoproxil fumarate and emtricitabine (TRUVADA®, Gilead), didanosine (VIDEX®, VIDEX EC® (extended release), Bristol Myers-Squibb), stauvudine (ZERIT®, Bristol Myers-Squibb), and abacavir (ZIAGEN®, GlaxoSmithKline), acyclovir, ddI, ddC, and d4T. Combinations of nucleoside reverse transcriptase inhibitors may also be employed.

The second active agent may comprise a non-nucleoside reverse transcriptase inhibitor. Suitable non-nucleoside reverse transcriptase inhibitors include (S)-6-chloro-4-(cyclopropylethynyl)-1,4-dihydro-4-(trifluoromethyl)-2H-3,1-benzoxazin-2-one (efavirenz) (Sustiva®, Bristol-Myers Squibb), 9-[(R)-2-[[bis[[(isopropoxycarbonyl)oxy]methoxy] phosphinyl]methoxy]propyl]adenine disoproxil fumarate (tenofovir) (VIREAD®, Gilead), and delaviridine (RESCRIPTOR®, Pfizer). Combinations of non-nucleoside reverse transcriptase inhibitors may also be employed.

The second active agent may comprise a protease inhibitor. Protease inhibitors interfere with a viral protease, an enzyme that cuts newly created protein chains into smaller proteins. Suitable protease inhibitors include, for example, amprenavir (Agenerase®, GlaxoSmithKline), fos-amprenavir (LEXIVA®, GlaxoSmithKline), GW-433908 (prodrug of Amprenavir, Glaxo/ Vertex), indinavir (Crixivan®, Merck), saquinavir (Fortovase®, Roche), saquinavir mesylate (INVERase®, Haffman-La Roche), nelfinavir (Viracept®, Pfizer), ritonavir (Norvir®, Abbott Laboratories), a blend of lopinavir and ritonavir (Kaletra®, Abbott Laboratories), atazanavir (Reyataz®, Bristol-Myers Squibb), tipranavir (APTIVUS®, Boehringer-Ingelheim), and combinations comprising one or more of the foregoing protease inhibitors.

The second active agent may comprise an integrase inhibitor. Integrase inhibitors prevent HIV from inserting its own genetic material into the host cell, so HIV cannot make new viruses. Several integrase inhibitors are currently being tested. These include GS 9137 and JTK 303 both from Gilead.

The second active agent may comprise a fusion inhibitor. Fusion inhibitors block HIV entry into cells by interfering with the fusion process of the virus and cell membrane. Certain fusion inhibitors block HIV entry into CD4 cells. One example of a suitable fusion inhibitor is enfuvirtide (FUZEON®, Hoffman-La Roche and Trimeris).

### COMBINATION BLVUCITABINE PHARMACEUTICAL COMPOSITIONS AND DOSAGE FORMS

Provided herein are pharmaceutical compositions and dosage forms comprising Elvucitabine and a second active agent, including pharmaceutical compositions suitable for low dosage and/or low frequency administration, comprising Elvucitabine or a salt thereof, a second active agent, and at least one pharmaceutically acceptable excipient such as a filler. The second active agent may comprise a single active chemical entity or more than one active chemical entity. Certain embodiments provided herein include pharmaceutical compositions in which Elvucitabine is combined with two additional active chemical entities, including embodiments in which the two additional active chemical entities are one additional nucleoside analog and either a non-nucleoside reverse transcriptase inhibitor or a protease inhibitor.

The pharmaceutical composition may be in a form suitable for administration to a human subject, for example, but is preferably an oral dosage form such as a tablet or capsule. The pharmaceutical compositions provided herein may be formulated by a variety of methods apparent to those of skill in the art of pharmaceutical formulation.

The pharmaceutical composition may be in the form of a once a day dosage form comprising Elvucitabine and a second active agent. Certain once a day dosage forms described herein comprise from about 2.5 mg to about 20 mg, or from about 2.5 to 15 mg, or about 2.5 mg to 10 mg, or an effective amount but not more than 6.5 mg, e.g. from 2.5 mg to not more than 6.5 mg Elvucitabine. In another embodiment, the pharmaceutical composition may be in the form of a once per 48 hours dosage form comprising Elvucitabine and a second active agent. In certain embodiments the once per 48 hours dosage form comprises about 5 mg to about 20 mg Elvucitabine, an effective amount but not more than 6.5 mg, e.g. from about 2.5 mg to not more than 6.5 mg, or about 10 mg, about 15 mg, or about 20 mg Elvucitabine. The pharmaceutical composition may also be in the form of a dosage form comprising Elvucitabine and a second active agent suitable for administration twice per week, a may comprise about 30 mg to about 50 mg Elvucitabine per dose. The pharmaceutical composition may be in the form of a once per week dosage form comprising Elvucitabine and a second active agent. Certain once per week dosage forms described herein comprise about 40 mg to about 100 mg Elvucitabine, for example once per week dosage forms comprising Elvucitabine and a second active agent may be comprised of about 40 mg, about 50 mg, about 75 mg, or about 100 mg Elvucitabine.

In certain embodiments, the Elvucitabine pharmaceutical compositions comprise silicified microcrystalline cellulose (SMCC). An exemplary silicified microcrystalline cellulose is PROSOLV 90, a product composed of about 98 percent microcrystalline cellulose and about 2 percent colloidal silicon dioxide. Silicification of the microcrystalline cellulose is achieved through a process that results is an intimate association of the colloidal silicon dioxide and microcrystalline cellulose. The median particle size of PROSOLV 90 silicified microcrystalline cellulose is about 90 µm. Another suitable silicified microcrystalline cellulose is a silicified microcrystalline cellulose having a median particle size of about 50 µm, e.g. PROSOLV 50.

The Elvucitabine pharmaceutical compositions may comprise pharmaceutically compatible excipients. Excipients may be added to facilitate manufacture, enhance stability, control release, enhance product characteristics, enhance bioavailability, enhance patient acceptability, etc. Pharmaceutical excipients include binders, disintegrants, lubricants, glidants, compression aids, colors, sweeteners, preservatives, suspending agents, dispersing agents, film formers, flavors, printing inks, etc. Excipients include inert diluents, such as calcium carbonate, sodium carbonate, mannitol, lactose, cellulose, and combinations comprising one or more of the foregoing diluents; binders such as starch, methylcellulose, gelatin, sucrose, and combinations comprising one or more of the foregoing binders; disintegrants such as starch, alginic acid, sodium starch glycolate, croscarmelose, and combinations comprising one or more of the foregoing disintegrants; and lubricants such as magnesium stearate, stearic acid, talc, and combinations comprising one or more of the foregoing lubricants. Agents such as potassium phosphate, magnesium oxide, potassium citrate, and sodium phosphate may also be present. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. The selection of excipients often depends on secondary considerations like taste, cost, and shelf stability.

In certain embodiments described herein the invention includes a pharmaceutical composition comprising Elvucitabine, lactose monohydrate, and anhydrous lactose. The pharmaceutical composition may also include other ingredients such as crospovidone, calcium silicate, and magnesium stearate.

In certain embodiment the lactose monohydrate and anhydrous lactose taken together comprise about 60 percent to about 90 percent of the Elvucitabine pharmaceutical composition by weight.

The invention provides a pharmaceutical composition wherein less than 2.0%, less than 1.5%, less than 1.0%, or less than 0.5% is magnesium stearate. The invention also provides a pharmaceutical composition containing Elvucitabine and magnesium stearate, wherein from about 0.05 to about 1 percent, or in some embodiments from about 0.10 to about 0.50 percent, or about 0.05 to about 0.50 percent, of the composition is magnesium stearate.

### METHODS OF TREATMENT

Provided herein is a method of treating a viral infection comprising administering to a patient suffering from a viral infection, for example an HIV or HBV infection, Elvucitabine and a second active agent, wherein the second active agent is an immunomodulatory compound, an antiviral agent, or a combination comprising one or more of the foregoing active agents, with the proviso that the antiviral agent is not interferon, a nucleoside, or a nucleoside analog. Alternatively the second active agent may be immunomodulatory compound, including interferon, or an antiviral agent, including a nucleoside and nucleoside analogue, or a combination comprising one or more of the foregoing active agents, wherein the dosage form contains an effective amount but not more than 6.5 mg Elvucitabine, typically from about 2.5 mg to not more than 6.5 mg Elvucitabine, and is not give more than once daily.

The second active agent may comprise more that one active chemical entity. Methods of treatment include methods in which Elvucitabine is combined with the second active agent in a single dosage form, methods in which Elvucitabine and the second active agent are packaged together, and methods in which Elvucitabine and the second active agent are prescribed together.

In one embodiment, the pharmaceutical composition comprising Elvucitabine and optionally a second active agent is administered once per day, and the dosage of Elvucitabine is 2.5 mg to 20 mg per day, or about 2.5 mg to 15 mg per day, or about 2.5 mg to 10 mg per day.

In another embodiment, the Elvucitabine pharmaceutical composition comprising Elvucitabine and optionally the second active agent is administered once every 48 hours. In certain embodiments about 10 mg, about 15 mg, or about 20 mg Elvucitabine may be administered together with a second active agent once per 48-hour interval.

In yet another embodiment, the pharmaceutical composition comprising Elvucitabine and optionally a second active agent is administered twice per week, and the Elvucitabine dosage is 30 mg to 50 mg per dose.

The method of treating a viral infection may comprise administering about 40 mg to about 100 mg Elvucitabine per week and a second active agent to a patient suffering from a viral infection is further provided. For example about 40 mg, about 50 mg, about 75 mg, or about 100 mg Elvucitabine may be administered once per week. In one embodiment, Elvucitabine is administered once every 2 to 7 days. In another embodiment, the pharmaceutical composition comprising Elvucitabine and optionally a second active agent is administered once per week, and the Elvucitabine dosage is 15 mg to 60 mg per week.

The Elvucitabine and the second active agent may be administered on the same or a different dosing schedule. In one embodiment, the Elvucitabine and the second active agent may be administered once per day. In another embodiment, the Elvucitabine may be administered once every 48 hours and the second active may be administered once per day. In yet another embodiment, the Elvucitabine may be administered once per week, and the second active agent may be administered once per day.

In one embodiment, the second active agent is administered once per day. When the second active agent comprises efavirenz, the efavirenz may be administered at 200 to 1000 mg/day, or 600 mg/day. When the second active agent is tenofovir, the tenofovir may be administered at 100 to 800 mg/day, or 300 mg/day.

In certain embodiments, administration may comprise administration of a loading dose. A loading dose, as used herein, is a quantity higher than the average or maintenance dose, used at the initiation of therapy to rapidly establish a desired level of the Elvucitabine. A loading dose may comprise about 5 to about 40 mg of Elvucitabine. Alternatively the loading dose may comprise a dosage of the second active agent, which is higher than the maintenance dose of the second active agent. The loading dose is followed by a maintenance dose of Elvucitabine and optionally a second active agent, which is lower than the loading dose.

In certain embodiments described herein the Elvucitabine and the second active agent are administered as an oral dosage form. Oral dosages of Elvucitabine may be in the form of a tablet or capsule, or other pharmaceutically acceptable form.

Also provided herein are methods of increasing patient compliance with anti-viral therapy, such as treatment of an HIV or HBV infection, by providing a dosage regimen in which Elvucitabine is administered together with a second active agent and the Elvucitabine is formulated for once daily, or less frequent administration.

Also included herein is a method of improving safety of treating a viral infection, for example an HIV or HBV infection comprising administering Elvucitabine and a second active agent to a patient suffering from a viral infection wherein the amount of Elvucitabine administered is: about 2.5 mg to about 20 mg per day, but preferably about 5 to 10 mg per day, for example about 5 mg, about 7 mg, or about 10 mg per day; or about 5 mg to about 20 mg Elvucitabine per 48 hour interval, for example about 10 mg, about 15 mg, or about 20 mg Elvucitabine per 48 hour interval; or about 40 mg to about 100 mg per week, for example 40 mg, about 50 mg, about 75 mg, or about 100 mg Elvucitabine per week.

Also included herein are methods of treating a viral infection, such as an HIV or HBV infection, comprising providing Elvucitabine and a second active agent, either in a single dosage form or in separate dosage forms, in a container, and instructions for using the Elvucitabine and second active agent to treat the viral infection.

Methods of treating a viral infection in a patient further include a method of providing an optimal blood concentration of Elvucitabine comprising administering a first amount Elvucitabine and a second active agent to the patient, monitoring the Cₘₐₓ of the Elvucitabine, adjusting the first amount of Elvucitabine so that the amount provides an Elvucitabine C*ₘₐₓ* of not more than 45 micrograms/ L. Methods of treating a viral infection in a patient also include a method of providing an optimal concentration of Elvucitabine comprising administering a first amount Elvucitabine and a second active agent to the patient, monitoring the Cₘₐₓ and Cₘᵢₙ of the Elvucitabine, adjusting the first amount of Elvucitabine so that the amount provides an Elvucitabine C*ₘₐₓ*: Cₘᵢₙ ratio of not more than 10: 1.

### DOSAGE FORMS CHARACTERIZED BY AUC OR CMAX

Elvucitabine forms for low dosage administration described herein exhibit characteristic plasma concentrations over time. When integrated the graph of plasma concentration over time provides a characteristic "area under the curve" or AUC.

Certain Elvucitabine dosage forms described herein exhibit an AUC at steady state for a 24 hour period of about 300 microgram hour/ liter (µg h/L).

Other embodiments included herein include an oral dosage form comprising Elvucitabine and a second active agent, wherein the second active agent is an immunomodulatory compound or an antiviral agent or a combination comprising one or more of the foregoing active agents and wherein the dosage form provides an Elvucitabine C*ₘₐₓ* of not more than 45 micrograms/ L. An oral dosage form comprising Elvucitabine and a second active agent that provides an Elvucitabine C*ₘₐₓ* of more than 2 micrograms/ L and less than 40 micrograms/ L is also included herein.

### EXEMPLARY PHARMACEUTICAL COMPOSITIONS

The low dosage pharmaceutical compositions provided herein may be formulated by a variety of methods apparent to those of skill in the art of pharmaceutical formulation. Suitable formulations include, for example, tablets, capsules, press coat formulations, easily administered formulations, etc.

### PREPARATION OF CORES

Cores comprising Elvucitabine can be prepared by various mixing, comminution and fabrication techniques readily apparent to those skilled in the area of drug formulations. Examples of such techniques are as follows:
(1) Direct compression, using appropriate punches and dies; the punches and dies are fitted to a suitable rotary tablet press;
(2) Injection or compression molding using suitable molds fitted to a compression unit; and
(3) Granulation followed by compression.

When cores are made by direct compression, the addition of lubricants may be helpful to promote powder flow and to reduce capping of the particle (breaking off of a portion of the particle) when the pressure is relieved. Useful lubricants include, for example, magnesium stearate in a concentration of about 0.05% to about 3% by weight, preferably less than about 1% by weight, in the powder mix. Additional excipients may be added to enhance powder flowability and reduce adherence.

Prior to compression, the Elvucitabine, filler, lubricant and additional excipients are blended for a time sufficient to produce the desired pressed product. Blending times may be about 15 to about 60 minutes.

Core shape can influence the coverage and stability of the coat. For example, sharp corners may lead to stress points on the coat and cause weaknesses in the structure possibly leading to premature release of active agent from the dosage form. Furthermore, areas of thin coating are susceptible to breaking and cracking and hence ineffective for sustained-release and taste masking.
Aspect ratio is a measure of the thickness to diameter. For example, ratio of about 1 is a box or sphere. A high aspect ratio may result in a relatively thin coat leading to a more rapid release rate of active agent than is preferred. A low aspect ratio spherical shape of the core is advantageous for both solubility of the coat and high payload of active agent. In some embodiments the invention provides a core comprising Elvucitabine and optionally a second active agent having an aspect ratio that is less than about 1, or about 0.5 to about 1, and or approximately about 0.5 to about 0.6.

### PREPARATION OF ELVUCITABINE CONTAINING SUBUNITS

Elvucitabine, the second active agent, and any optional additives may be prepared in many different ways, for example as subunits. Pellets comprising an active agent can be prepared, for example, by a melt pelletization technique. In this technique, the active agent in finely divided form is combined with a binder and other optional inert ingredients, and thereafter the mixture is pelletized, e.g., by mechanically working the mixture in a high shear mixer to form the pellets (e.g., pellets, granules, spheres, beads, etc., collectively referred to herein as "pellets"). Thereafter, the pellets can be sieved in order to obtain pellets of the requisite size. The binder material may also be in particulate form and has a melting point above about 40 oC.

Subunits, e.g., in the form ofmultiparticulates, can be compressed into an oral tablet using conventional tableting equipment using standard techniques. Alternatively, subunits may be in the form of micro-tablets enclosed inside a capsule, e.g., a gelatin capsule. For this, a gelatin capsule as is employed in pharmaceutical formulations can be used, such as the hard gelatin capsule known as CAPSUGEL, available from Pfizer.

### PARTICLES

Some oral dosage forms described herein contain Elvucitabine and optionally a second active agent in the form of particles. Such particles may be compressed into a tablet, present in a core element of a coated dosage form, such as a taste masked dosage form, a press coated dosage form, or an enteric coated dosage form, or may be contained in a capsule, osmotic pump dosage form, or other dosage form.

For particles, such as powder particles, present in the core element of a coated dosage form, the core element may have a particle size distribution with a median of about 100 µm. The particles in the distribution may vary from about 1 µm to about 250 µm, more preferably from 25 µm to about 250 µm, most preferably about 35 µm to about 125 µm. If the median of the distribution is close to either extreme of the distribution, the taste masking or sustained-release characteristics may be affected. In a particle size range of about 25 µm to about 250 µm, no more than about 25% of particles can be less than about 25 µm, and no more than about 25% can be over about 250 µm.

Inconsistencies in size and shape can lead to inconsistent coating. Where the particles containing active agent are of different size and shape, polymeric coating materials such as ethyl cellulose may deposit differently on each particle. It is therefore preferable for coated dosage forms that substantially all particles of the dosage form have substantially the same size and shape so that the coating process is better controlled and maintained.

### TABLETS AND CAPSULES

Particular embodiments provided herein include capsules or tablets comprising from about 2.5 to about 20 mg Elvucitabine and optionally a second active agent, formulated for daily administration, preferably about 5 mg to about 10 mg for daily administration, from about 10 mg to about 20 mg for administration once every 48 hour interval, and from about 40 mg to about 100 mg for once weekly administration, In other embodiments, the invention includes capsules and tablets suitable for once daily administration comprising Elvucitabine, and optionally a second active agent, containing an effective amount but not more than 6.5 mg Elvucitabine, e.g. from about 2.5 mg to not more than 6.5 mg Elvucitabine.

Pharmaceutical compositions for use in tablets may have certain desirable physical properties. For example the invention provides a pharmaceutical composition comprising Elvucitabine and optionally a second active agent having a tensile strength greater than 2100 KPa, or in other embodiments having a tensile strength of about 2200 KPa to about 7700 KPa. Also provided is pharmaceutical composition comprising Elvucitabine and optionally a second active agent having a compression range of about 80 MPa to about 350 MPa .The invention also provides an oral dosage form of Elvucitabine and optionally a second active agent having a hardness greater than 7 KGF, or in other environments having a hardness of about 5.5 to about 45 KGF, specifically about 5 KGF to about 35 KGF. The invention also provides an oral dosage form comprising Elvucitabine and optionally a second active agent having a hardness greater than 9 KGF to 21 KGF for 5 mg strength tablets and 16 KGF to 34 KGF for 10 mg strength tablets.

The invention includes an oral dosage form, for example a tablet, comprising Elvucitabine and optionally a second active agent in a core, wherein the core has an aspect ratio of 0.50 to 0.55. The invention includes a coated table comprising Elvucitabine and optionally a second active agent, wherein the coated tablet has an aspect ratio of from 0.51 to 0.56.

The Elvucitabine and optionally a second active agent, optionally in the form of pellets, may be disposed in a capsule. The capsule may comprise a hard capsule and/or soft capsule. A hard capsule may be composed of two parts, a cap and a body, which are fitted together after the larger body is filled with the active agent. The hard capsule may be fitted together by slipping or telescoping the cap section over the body section, thus completely surrounding and encapsulating the active agent. A soft capsule may be a one-piece soft capsule of sealed construction encapsulating the active agent. The soft capsule may be made by various processes, such as the plate process, the rotary die process, the reciprocating die process, and the continuous process. A gelatin capsule as is employed in pharmaceutical formulations can be used, such as the hard gelatin capsule known as CAPSUGEL, available from Pfizer.

### COATINGS

The pharmaceutical compositions described herein may be coated with a functional or non-functional coating.

In one embodiment, a functional coating comprises an enteric coating comprising an enteric polymer. The enteric polymer should be non-toxic and is predominantly soluble in the intestinal fluid, but substantially insoluble in the gastric juices. Examples include polyvinyl acetate phthalate (PVAP), hydroxypropylmethyl-cellulose acetate succinate (HPMCAS), cellulose acetate phthalate (CAP), methacrylic acid copolymers, hydroxy propyl methylcellulose succinate, cellulose acetate succinate, cellulose acetate hexahydrophthalate, hydroxypropyl methylcellulose hexahydrophthalate, hydroxypropyl methylcellulose phthalate (HPMCP), cellulose propionate phthalate, cellulose acetate maleate, cellulose acetate trimellitate, cellulose acetate butyrate, cellulose acetate propionate, methacrylic acid/methacrylate polymer (acid number 300 to 330 and also known as EUDRAGIT L), which is an anionic copolymer based on methacrylate and available as a powder (also known as methacrylic acid copolymer, type A NF, methacrylic acid-methyl methacrylate copolymer, ethyl methacrylate-methylmethacrylate-chlorotrimethylammonium ethyl methacrylate copolymer, and the like, and combinations comprising one or more of the foregoing enteric polymers. Other examples include natural resins, such as shellac, SANDARAC, copal collophorium, and combinations comprising one or more of the foregoing polymers. Yet other examples of enteric polymers include synthetic resin bearing carboxyl groups. A suitable methacrylic acid copolymer is commercially available as Acryl EZE®. The methacrylic acid: acrylic acid ethyl ester 1:1 copolymer solid substance of the acrylic dispersion sold under the trade designation "EUDRAGIT L-100-55" maybe suitable.

The enteric coating may comprise about 5 wt% to about 25 wt%, or about 8 wt% to about 16 wt% of the total weight of the dosage form.

The dosage forms may comprise additional functional and nonfunctional coatings such as seal coats and overcoats. The coating material may include a polymer, preferably a film-forming polymer, for example, methyl cellulose, ethyl cellulose, hydroxypropyl cellulose, hydroxypropyl methyl cellulose, hydroxybutyl methyl cellulose, cellulose acetate, cellulose propionate (lower, medium or higher molecular weight), cellulose acetate propionate, cellulose acetate butyrate, cellulose acetate phthalate, carboxymethyl cellulose, cellulose triacetate, cellulose sulphate sodium salt, poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene)high density, (poly propylene), poly (ethylene glycol poly (ethylene oxide), poly (ethylene terephthalate), poly(vinyl alcohol), poly(vinyl isobutyl ether), poly(vinyl acetate), poly (vinyl chloride), polyvinyl pyrrolidone, and combinations comprising one or more of the foregoing polymers. A suitable hydroxypropyl methylcellulose is commercially available as OPADRY® from Colorcon.

A seal coat and/or and overcoat may comprise about 1 wt% to about 3 wt% of the total weight of the dosage form.

The inclusion of an effective amount of a plasticizer in the coating composition may improve the physical properties of the film. Generally, the amount of plasticizer included in a coating solution is based on the concentration of the polymer, e.g., most often from about 1 to about 50 percent by weight of the polymer. Concentrations of the plasticizer, however, can be determined by routine experimentation.

Examples of plasticizers for ethyl cellulose and other celluloses include plasticizers such as dibutyl sebacate, diethyl phthalate, triethyl citrate, tributyl citrate, triacetin, and combinations comprising one or more of the foregoing plasticizers, although it is possible that other water-insoluble plasticizers (such as acetylated monoglycerides, phthalate esters, castor oil, etc.) can be used.

Examples of plasticizers for acrylic polymers include citric acid esters such as triethyl citrate 21, tributyl citrate, dibutyl phthalate, 1,2-propylene glycol, polyethylene glycols, propylene glycol, diethyl phthalate, castor oil, triacetin, and combinations comprising one or more of the foregoing plasticizers, although it is possible that other plasticizers (such as acetylated monoglycerides, phthalate esters, castor oil, etc.) can be used.

Suitable methods are used to apply the coating to tablet cores, for example. Processes such as simple or complex coacervation, interfacial polymerization, liquid drying, thermal and ionic gelation, spray drying, spray chilling, fluidized bed coating, pan coating, electrostatic deposition, may be employed. A substantially continuous nature of the coating may be achieved, for example, by spray drying from a suspension or dispersion of active agent in a solution of the coating composition including a polymer in a solvent in a drying gas having a low dew point.

When a solvent is used to apply the coating, the solvent is may be water or an organic solvent. The solvent may constitute a good solvent for the coating material, but is substantially a non-solvent or poor solvent for an active agent. The solvent may be selected from water, alcohols such as methanol, ethanol, halogenated hydrocarbons such as dichloromethane (methylene chloride), hydrocarbons such as cyclohexane, and combinations comprising one or more of the foregoing solvents.

The concentration of polymer in the solvent will normally be less than about 75 wt%, and typically about 10 wt% to about 30 wt%. After coating, the coated dosage forms may be allowed to cure for about 1 to about 2 hours at a temperature of about 50°C to about 60°C, more preferably of about 55°C.

The coatings may be about 0.005 µm to about 25 µm thick, preferably about 0.05 µm to about 5 µm.

### PACKAGED PHARMACEUTICAL COMPOSITIONS

Packaged pharmaceutical compositions comprising an Elvucitabine pharmaceutical composition and a second active agent in a container and instructions for using the packaged pharmaceutical composition for treating a viral infection, such as an HIV infection, by administering about 2.5 to about 10 mg Elvucitabine per day, or treating a patient having a viral infection by administering about 5 mg to about 20 mg Elvucitabine per 48 hour interval, preferably about 10 mg to about 20 mg Elvucitabine per 48 hour interval, or about 40 mg to about 100 mg Elvucitabine per week to a patient suffering from a viral infection are further provided herein. Elvucitabine and the second active agent in the packaged pharmaceutical composition may be combined in a single dosage form or present in separate dosage forms.

Typically the instructions will be instructions for using the pharmaceutical composition to treat an HBV or HIV infection. Packaged pharmaceutical compositions in which the Elvucitabine is present as an oral dosage form are disclosed herein.

The invention includes providing prescribing information, for example, to a patient or health care provider, or as a label in a packaged pharmaceutical composition. Prescribing information may include for example efficacy, dosage and administration, contraindication and adverse reaction information pertaining to the pharmaceutical composition.

### EXAMPLES

The following examples further illustrate the invention but, of course, should not be construed as in any way limiting its scope.

### ABBREVIATIONS

The following abbreviations are used in the examples, which follow, and elsewhere. This list in not meant to be an all-inclusive list of abbreviations used in the application as additional standard abbreviations, which are readily understood by those skilled in the art of pharmaceutical compositions, may also be used.

| | |
|---|---|
| CFM | Cubic Feet per Minute |
| CU | Content Uniformity |
| h | hour |
| KGF | Kilogram force |
| KPa | Kilo Pascal |
| kp | kilopond (kg/mm²) = 9.807 Pa |
| MPa | Milli Pascal |
| NMT | Not more than |
| RSD | Relative Standard Deviation |
| TEC | Triethylcitrate |

### EXAMPLE 1. Low DOSE ELVUCITABINE TABLETS

Elvucitabine exhibits adhesion (to metal surfaces) and limited compactability. Typical excipients used to resolve such issues in a direct blend are not preferable based on the excipient compatibility results. Excipients that increase compactability, decrease adhesion, and are not acidic are desirable to address processing challenges.

All tablets are compressed using ¼" standard concave tooling.

The following 5 mg dosage form is made at a 150 gram batch size and large enough to run a few rotations on the automated press. The 20 mg dosage form is made at smaller batch sizes (40 grams) to conserve drug, and therefore is not evaluated under full conditions of an automated press. However the tablets made from hand turning of the wheel resulted in tablets with good surfaces, hardness and friability. The 5 mg tablets pass content uniformity specifications (range: 85% - 115%; %RSD </= 6%) with CU= 87.8% and %RSD = 1.7.

**Table 4.**

| **Component** | **%** | **mg/tablet** | **g/1000 tablets** |
|---|---|---|---|
| Elvucitabine | 3.33 | 5 | 5.0 |
| Lactose (Fast Flo) | 45.21 | 67.815 | 67.815 |
| Lactose (Anhydrous - Direct tableting Grade) | 45.21 | 67.815 | 67.815 |
| Crospovidone | 3 | 4.5 | 4.5 |
| Calcium Silicate | 2 | 3.0 | 3.0 |
| Magnesium Stearate | 1.25 | 1.875 | 1.875 |
| TOTALS | 100% | 150 mg | 150g |
| Hardness: 5.3 Kp, Friability: 0.34%, no cappping | | | |

**Table 5.**

| **Component** | **%** | **mg/tablet** | **g/200 tablets** |
|---|---|---|---|
| Elvucitabine | 10 | 20 | 4.0 |
| Lactose (Fast Flo) | 41.875 | 83.75 | 16.75 |
| Lactose (Anhydrous - Direct tableting Grade) | 41.875 | 83.75 | 16.75 |
| Crospovidone | 3 | 6 | 1.2 |
| Calcium Silicate | 2 | 4 | 0.8 |
| Magnesium Stearate | 1.25 | 2.5 | 0.5 |
| TOTALS | 100% | 200 mg | 40g |
| Hardness: 5.9 Kp, Friability: 0%, no cappping | | | |

**Table 6.**

| **Component** | **%** | **mg/tablet** | **g/200 tablets** |
|---|---|---|---|
| Elvucitabine | 25 | 50 | 10 |
| Lactose (Fast Flo) | 34.375 | 68.75 | 13.75 |
| Lactose (Anhydrous - Direct tableting Grade) | 34.375 | 68.75 | 13.75 |
| Crospovidone | 3 | 6 | 1.2 |
| Calcium Silicate | 2 | 4 | 0.8 |
| Magnesium Stearate | 1.25 | 2.5 | 0.5 |
| TOTALS | 100% | 200 mg | 40g |
| Hardness: 4.5Kp, Friability: 0%, no capping | | | |

Two additional batches are made for the 5 mg and 20 mg dose with an additional blending step. Content uniformity results are also presented below. The extra blending step results in a more optimum content uniformity. The 5 mg and 20 mg dose are formulated to a total tablet weight of 150mg and the 50 mg dose is tableted to a total tablet weight of 200 mg.

**Table 7.**

| | **5 mg dose** | | **20 mg dose** | |
|---|---|---|---|---|
| Component | % | mg/tablet | % | mg/tablet |
| Elvucitabine | 3.33 | 5.0 | 13.33 | 20.00 |
| Lactose (Fast Flo) | 45.21 | 67.81 | 40.21 | 60.32 |
| Lactose (Anhydrous - Direct tableting Grade) | 45.21 | 67.81 | 40.21 | 60.32 |
| Crospovidone | 3.00 | 4.50 | 3.00 | 4.50 |
| Calcium Silicate | 2.00 | 3.00 | 2.00 | 3.00 |
| Magnesium Stearate | 1.25 | 1.88 | 1.25 | 1.87 |
| TOTALS | 100 | 150 | 100 | 150 |

**Table 8.**

| **Content Uniformity Data** | | |
|---|---|---|
| | % CU | % RSD |
| 5 mg dose | 89.4 | 4.6 |
| 20 mg dose | 93.2 | 4.4 |

### EXAMPLE 2. COATED TABLETS

Coated tablets are prepared with the application of a "base-coat" of hydroxypropylmethylcellulose (OpaDry). This coating agent smoothes tablet surface imperfections, providing a suitable surface for adhesion of the enteric coat.

**Table 9.**

| | |
|---|---|
| Core | Elvucitabine (20 mg) Tablets |
| Load | 100 tabs 15.033 g |
| Coating System | Sureteric (15% dispersion) |
| Target Coating Level | 14% |
| Coated Product | (20 mg) Enteric Coated Tablets (Sureteric) |

| | |
|---|---|
| Coated tablets appear to have a uniform, slightly rough, off-white coat. Six tablets tested in 0.1 N HCl - all passed. | |

**Table 10.**

| | |
|---|---|
| Core | Elvucitabine (20 mg) Tablets |
| Load | 100 tabs 15.031 g |
| | Eudragit L30 D55 with 10% |
| Coating System | Triethylcitrate |
| Target Coating Level | 14% |
| Coated Product | (20 mg) Enteric Coated Tablets (Eudragit) |

| | |
|---|---|
| Acceptable tablets obtained - slightly rough surface with glossy finish. Six tablets tested in 0.1 N HCl - all passed. | |

**Table 11.**

| | |
|---|---|
| Core | Elvucitabine (20 mg) Tablets |
| Load | 100 tabs 15.01 g Opadry followed by Eudragit L30 D55 with 2% |
| Coating System | TEC |
| Target Coating Level | 14% (2% Opadry/12% Eudragit) |
| | ACH 126,443 (20 mg) Enteric Coated Tablets |
| Coated Product | (Opadry/Eudragit) |

| | |
|---|---|
| 15% dispersion of Opadry Clear (YS-1-7472) Opadry coat = excellent, glossy surface. | |

### EXAMPLE 3. ADDITIONAL COATED TABLETS

The following pharmaceutical compositions are prepared by blending the first five ingredients, Elvucitabine, lactose fast flow, lactose anhydrous, crospovidone, and calcium silicate for 13 minutes. Magnesium stearate is then added. Pharmaceutical compositions having more that 0.5 percent magnesium stearate by weight are blended approximately 3 minutes longer. Pharmaceutical compositions containing about 0.5% magnesium stearate or less are blended and additional 2,0 - 25 minutes. Tableting is performed as described in Example 1 and the enteric and final coat are applied.

**Table 12.**

| **Ingredient 2.5 mg Strength** | **mg/ tablet** | **%** | **mg/ tablet** | **%** |
|---|---|---|---|---|
| Elvucitabine | 2.5 | 3.33 | 2.5 | 3.33 |
| Lactose fast flow | 36.626 | 44.835 | 34.406 | 45.785 |
| Lactose Anhydrous | 36.626 | 44.835 | 34.406 | 45.785 |
| Crospovidone | 4.5 | 3.00 | 4.5 | 3.00 |
| Calcium Silicate | 3.0 | 2.00 | 3.0 | 2.00 |
| Magnesium Stearate | 3.0 | 2.00 | 0.15 | 0.10 |
| Total | 75.0 | 100.0 | 75.0 | 150.0 |

**Table 13.**

| Ingredient 5 mg Strength | mg/ tablet | % | mg/tablet | % |
|---|---|---|---|---|
| Elvucitabine | 5.0 | 3.33 | 5.0 | 3.33 |
| Lactose fast flow | 67.25 | 44.835 | 68.678 | 45.785 |
| Lactose Anhydrose | 67.25 | 44.835 | 68.678 | 45.785 |
| Crospovidone | 4.5 | 3.00 | 4.5 | 3.00 |
| Calcium Silicate | 3.0 | 2.00 | 3.0 | 2.00 |
| Magnesium Stearate | 3.0 | 2.00 | 0.15 | 0.10 |
| Total | 150.0 | 100.0 | 150.0 | 150.0 |

**Table 14.**

| **Ingredient 20 mg Strength** | **mg/ tablet** | **%** | **mg/ tablet** | **%** |
|---|---|---|---|---|
| Elvucitabine | 20.0 | 13.33 | 20.0 | 13.33 |
| Lactose fast flow | 59.75 | 39.83 | 61.095 | 40.73 |
| Lactose Anhydrose | 59.75 | 39.83 | 61.095 | 40.73 |
| Crospovidone | 4.5 | 3.00 | 4.5 | 3.00 |
| Calcium Silicate | 3.0 | 2.00 | 3.0 | 2.00 |
| Magnesium Stearate | 3.0 | 2.00 | 0.30 | 0.20 |
| Total | 150 | 100 | 150 | 100 |

**Table 15.**

| **Ingredient 50 mg Strength** | **mg/ tablet** | **%** | **mg/ tablet** | **%** |
|---|---|---|---|---|
| Elvucitabine | 50.0 | 25.0 | 50.0 | 25.0 |
| Lactose fast flow | 68.0 | 34.0 | 69.85 | 34.925 |
| Lactose Anhydrose | 68.0 | 34.0 | 69.85 | 34.925 |
| Crospovidone | 6.0 | 3.00 | 3.00 | 3.00 |
| Calcium Silicate | 4.0 | 2.00 | 2.00 | 2.00 |
| Magnesium Stearate | 4.0 | 2.00 | 0.225 | 0.15 |
| Total | 150 | 100 | 150 | 100 |

**Table 16.**

| Ingredient 5 mg Strength | mg/ tablet | % |
|---|---|---|
| Elvucitabine | 5.0 | 3.33 |
| Lactose fast flow | 68.37 | 45.58 |
| Lactose Anhydrose | 68.37 | 45.58 |
| Crospovidone | 4.5 | 3.00 |
| Calcium Silicate | 3.0 | 2.00 |
| Magnesium Stearate | 0.75 | 0.50 |

**Table 17.**

| Tablet Coating System | | Dispersion Concentration | % Tablet weight gain |
|---|---|---|---|
| Base coat | Opadry II | 15% | 2 |
| Enteric coat | Eudragit L30 D55/TEC | 28.5% / 5% | 12 |

### EXAMPLE 4. MICRONIZATION OF ELVUCITABINE OR A SECOND ACTIVE AGENT

In certain embodiments described herein, Elvucitabine, or the second active agent used in a pharmaceutical composition may be micronized prior to tablet core manufacture. For example, micronization may be via a jet-mill process using a MICRONETTE M300 available from NUOVA GUSEO (Villanova sull'Arda-PC-Italy). Parameters are as follows: Injection pressure, 5 kg/cm²; micronization pressure, 9 kg/cm²; and cyclone pressure, 2.5 kg/cm². Capacity of micronization is 16 kg/h. Particle size may be determined by laser light scattering using a GALAI CIS 1 laser instrument (GALAI, Haifa, Israel), or by sieve analysis, for example. (See U.S. Patent No. 6,852,737 at Col. 29, lines 31-45, which is hereby incorporated by reference for its teachings regarding micronization of an active pharmaceutical ingredient.)

The particle sized reduction of Elvucitabine and optionally a second active agent using a micronization process may involve an eight inch spiral jet mill. The size reduction may be achieved using nitrogen gas at the following pressure: Mill pressure, 10 psi and Venturi pressure, 20 psi. The material may be fed into the mill at a rate of 12 kg/hour.

Alternatively, Elvucitabine may first be ground using a knife-grinder and then micronized in an ALJET Micronizer (FLUID ENERGY ALJET, Plumsteadsville, Pa., USA) using a pressurized dry nitrogen stream. (See U.S. Patent No. 6,555,156, at Col. 6, lines 13-18, which is hereby incorporated by reference for its teachings regarding micronization of an active pharmaceutical ingredient).

The mean particle size of the Elvucitabine after micronization is substantially all particles below 200 microns, or 90% of particles between 50 and 150 microns, or 98% of the particles between 25 and 180 microns.

### EXAMPLE 5. ELVUCITABINE COMMON BLEND FOR ENTERIC COATED TABLETS

Blend components are added according to Table 18.

**Table 18.**

| **Blend Component** | **Weight** | **% w/w** |
|---|---|---|
| Silicified Microcrystalline Cellulose, NF (Prosolv 90) | 10.645 kg | 92.57 |
| Sodium Starch Glycolate, NF, EP | 0.230 kg | 2.00 |
| Dipotassium Phosphate Powder, USP, PE | 0.230 kg | 2.00 |
| Magnesium Stearate, NF, EP (VG) | 0.012 kg | 0.10 |
| Elvucitabine, micronized | 0.383 kg | 3.33 |
| Totals | 11.5 kg | 100.0 |

Prosolv 90, micronized Elvucitabine, and Dipotassium Phosphate Powder, are added sequentially into a Bohle Bin Blender (BL07C, Warminster, Pennsylvania, USA) and blended for 10 ± 0.1 minutes at 11 ± 1 rpm. Additional Prosolv 90, Sodium Starch Glycolate, NF, EP, and Magnesium Stearate, NF, EP (VG) are added and blended for 10 ± 0.1 minutes at 11 ± 1 rpm. The material is then milled in a Bohle In-Line High Speed Mill (ML19) or equivalent and then passed through a 0.5 mm screen (35 Mesh) operated at a speed of 1400 rpm ± 50 rpm. Additional Prosolv 90 is passed through a Bohle In-Line High Speed Mill (ML19) and then passed through a 0.5 mm Screen (35 Mesh) operated at a speed of 1400 ± 50 rpm.

The 40 liter Bohle Bin Blender (BL07C) is charged with the milled materials. The blender bin cover is secured and the components are blended for about 60 minutes at 11 ± 1 rpm. Particle size analysis and bulk density analysis may be performed on the blend.

### EXAMPLE 6. PREPARATION OF ENTERIC COATED ELVUCITABINE TABLETS (5 MG STRENGTH)

### Example 6a. Manufacture of Tablet Cores

A 11.500 kg Elvucitabine common blend, prepared as described in Example 2, is loaded into a tablet compressing machine, such as a Fette 1200 B Tool Tablet Press (TP06) or equivalent, and tablets are formed using 0.2756" round plain upper and lower punches. Tablets are obtained having an average tablet weight of 150.0 mg with average acceptable upper and lower tablet weight limits of ± 5.0% (142.5 mg to 157.5 mg) and individual upper and lower tablet weight limits of ± 10% (135.0 mg to 165 mg).

Friability is determined by Current USP <1216> at the beginning and end of each compression run and is NMT 0.5%. The above tablet compression process yields 150.0 mg tablets ± 5.0 % having an average thickness of 3.67 mm ± 5% and an average hardness of 19.5 kp, with an average tablet hardness limit of from 17.0 kp to 22.0 kp.

Disintegration times are determined using Current USP <701> at the beginning and end of each compression batch. Disintegration time is NMT 5 minutes.

A 10 mg tablet core may be prepared similarly, but with a larger tablet punch.

### Example 6b. Coating of Elvucitabine Tablets

A seal coat, and enteric coat and an overcoat are applied according to Table 19.

**Table 19.**

| **Component** | **5 mg** | | **10 mg** | |
|---|---|---|---|---|
| | **%w/w** | **mg/tablet** | **%w/w** | **mg/tablet** |
| Tablet cores | 86.96 | 150 | 90.5 | 300 |
| Opadry Clear, seal coat | 2.18 | 3.75 | 1.81 | 6.0 |
| Acryl EZE beige, enteric coat | 10.43 | 18 | 7.24 | 24.0 |
| Opadry clear, over coat | 0.43 | 0.75 | 0.45 | 1.5 |
| Total | 100 | 172.5 | 100 | 331.5 |

The seal coat comprising OPADRY® Clear, the enteric coating comprising Acryl EZE®, and the over coat comprising OPADRY® clear applied sequentially as aqueous coating suspensions using a coating pan. The tablet cores are preheated to 46°C (Exhaust air temperature). The pan speed is adjusted to provide adequate tablet flow and the coating suspensions are sprayed onto the tablets at an atomizing air pressure of 18 - 30 psi; an inlet air temperature of 60 - 70°C for the seal coat and over coat, and of 42 - 50°C for the enteric coat; an exhaust air temperature of 40 to 50°C for the seal coat and over coat and 30 to 35°C for the enteric coat; a spray rate of 15 to 50 ml/ min.; and an inlet air flow of 175 to 300 CFM. One of skill in the would understand that the processing parameters for coating are dependent in part upon the size of the batch to be coated and can be adjusted accordingly.

### EXAMPLE 7. ADDITIONAL ELVUCITABINE COMMON BLENDS

**Table 20.**

| **Blend Component** | **Weight** | **% w/w** | **Weight** | **% w/w** |
|---|---|---|---|---|
| Silicified Microcrystalline Cellulose, NF (Prosolv 90) | 9.650 kg | 83.91 | 10.646 kg | 92.57 |
| Lactose Monohydrate | 0.995 kg | 8.66 | - | - |
| Sodium Starch Glycolate, NF, EP | 0.230 kg | 2.00 | 0.230 kg | 2.00 |
| Dipotassium Phosphate Powder, USP, PE | 0.230 kg | 2.00 | 0.230 kg | 2.00 |
| Magnesium Stearate, NF, EP (VG) | 0.012 kg | 0.10 | 0.012 kg | 0.10 |
| Elvucitabine, micronized | 0.383 kg | 3.33 | 0.383 kg | 3.33 |
| Totals | 11.5 kg | 100.0 | 11.5 kg | 100.0 |

**Table 21.**

| **Blend Component** | **Weight** | **% w/w** | **Weight** | **% w/w** |
|---|---|---|---|---|
| Silicified Microcrystalline Cellulose, NF (Prosolv 90) | 10.637 kg | 92.5 | 10.068 kg | 87.55 |
| Sodium Starch Glycolate, NF, EP | 0.201 kg | 1.75 | 0.345 kg | 3.00 |
| Dipotassium Phosphate Powder, USP, PE | 0.345 kg | 3.00 | 0.460 kg | 4.00 |
| Magnesium Stearate, NF, EP (VG) | 0.029 kg | 0.25 | 0.052 kg | 0.45 |
| Elvucitabine, micronized | 0.288 kg | 2.50 | 0.575 kg | 5.0 |
| Totals | 11.5 kg | 100.0 | 11.5 kg | 100.0 |

**Table 22.**

| Ingredient 5 mg Strength | mg/ tablet | % |
|---|---|---|
| Elvucitabine | 5.0 | 3.33 |
| Lactose fast flow | 68.37 | 45.58 |
| Lactose Anhydrose | 68.37 | 45.58 |
| Crospovidone | 4.5 | 3.00 |
| Calcium Silicate | 3.0 | 2.00 |
| Magnesium Stearate | 0.75 | 0.50 |

**Table 23.**

| Tablet Coating System | | Dispersion Concentration | % Tablet weight gain |
|---|---|---|---|
| Base coat | Opadry II | 15% | 2 |
| Enteric coat | Eudragit L30 D55/TEC | 28.5%/5% | 12 |

### EXAMPLE 8. ELVUCITABINE AND TENOFOVIR TABLET

Tablets containing elvucitabine and tenofovir can be formed using the protocol of Example 1, according to the following table:

**Table 24.**

| **Component** | **%** | **mg/TAB** | **g/1000 TABs** |
|---|---|---|---|
| Elvucitabine | 0.5 | 5 | 5.0 |
| Tenofovir | 30 | 300 | 300 |
| Lactose (Fast Flo) | 31.6 | 316.25 | 316.25 |
| Lactose (Anhydrous - Direct tableting Grade) | 31.6 | 316.25 | 316.25 |
| Crospovidone | 3 | 30 | 30 |
| Calcium Silicate | 2 | 20.0 | 20 |
| Magnesium Stearate | 1.25 | 12.5 | 12.5 |
| TOTALS | 100% | 1000 mg | 1000 g |

### EXAMPLE 9. ELVUCITABINE AND EFAVIRENZ TABLET

Tablets containing Elvucitabine and efavirenz can be formed using the protocol of Example 1, according to the following table:

**Table 25.**

| **Component** | **%** | **mg/TAB** | **g/1000 TABs** |
|---|---|---|---|
| Elvucitabine | 0.5 | 5 | 5.0 |
| Efavirenz | 30 | 600 | 300 |
| Lactose (Fast Flo) | 16.6 | 166.25 | 166.25 |
| Lactose (Anhydrous - Direct tableting Grade) | 16.6 | 166.25 | 166.25 |
| Crospovidone | 3 | 30 | 30 |
| Calcium Silicate | 2 | 20.0 | 20 |
| Magnesium Stearate | 1.25 | 12.5 | 12.5 |
| TOTALS | 100% | 1000 mg | 1000 g |

### EXAMPLE 10. TENOFOVIR TABLET

**Table 26.**

| **Component** | | **%w/w** | **Per unit content (mg/tablet)** |
|---|---|---|---|
| Tenofovir | | 34.0 | 150 |
| Lactose monohydrate NF | | | |
| | Extragranular | 54.0 | 238.4 |
| | Intragranular | 2.0 | 8.8 |
| Pregelatinized starch, NF | | 5.0 | 22.0 |
| Crosscarmellose , NF | | | |
| | Extragranular | 2.0 | 8.8 |
| | Intragranular | 2.0 | 8.8 |
| Magnesium stearate, NF | | 1.0 | 4.4 |

In the pharmaceutical composition, pregelatinized starch NF is used as a binder and disintegrant suitable for tablet compression. Croscarmellose sodium NF, which is internally cross-linked sodium carboxymethylcellulose, is used to facilitate tablet disintegration and dissolution. Lactose monohydrate NF is used as a diluent to aid manufacturing and to facilitate tablet dissolution. Magnesium stearate NF is used as a lubricant to facilitate tablet ejection from the tablet compression process.

Tablets containing tenofovir are made by blending pregelatinized starch, croscarmellose sodium and lactose monohydrate in a blender. Water is added until a suitable wet granulation is formed. The wet granulation is milled, dried in a fluid bed dryer to a moisture content of not more than 3% loss on drying, and the dried granules are passed through a mill. The milled granules are combined with extragranular excipients, croscarmellose sodium and lactose monohydrate, and blended in a mixer to obtain a powder blend. The powder blend is then blended with magnesium stearate and then compressed into tablets.

### EXAMPLE 11. EFAVIRENZ TABLET

The efavirenz, sodium starch glycolate and microcrystalline cellulose are granulated using an aqueous solution of sodium lauryl sulfate. This wet mass may then be dried in a fluid bed, tray or other suitable dryer. The dried granulation may then be milled to achieve the desired particle size distribution. This blend is compressed into tablets. These tablets may be coated if desired.

**Table 27.**

| **Component** | **Amount per Tablet, mg** | **w/w%** |
|---|---|---|
| Efavirenz | 300 | 50.00 |
| Sodium lauryl sulfate | 12 | 2 |
| Microcrystalline cellulose | 120 | 20 |
| Sodium starch glycolate | 120 | 20 |
| Lactose, hydrous | 42 | 7 |
| Magnesium stearate | 6 | 1 |
| TOTAL | 600 | |

1. A pharmaceutical composition comprising Elvucitabine and a second active agent, wherein the second active agent is an immunomodulatory compound, an antiviral agent, or a combination comprising one or more of the foregoing active agents, with the proviso that the second active agent is not interferon, a nucleoside or a nucleoside analog.
2. The pharmaceutical composition of Clause 1, wherein the antiviral agent is a tyrosine kinase inhibitor, a CCR5 inhibitor, a non-nucleoside reverse transcriptase inhibitor, a protease inhibitor, a fusion inhibitor, or an integrase inhibitor.
3. The pharmaceutical composition of Clause 1, wherein the composition additionally comprises one or more pharmaceutically acceptable excipients.
4. The pharmaceutical composition of Clause 1, in the form of a once a day dosage form comprising from about 2.5 mg to about 10 mg Elvucitabine.
5. The pharmaceutical composition of Clause1, in the form of a once per 48 hours dosage form comprising about 5 to about 20 mg of Elvucitabine.
6. The pharmaceutical composition of Clause2, wherein the non-nucleoside reverse transcriptase inhibitor comprises nevirapine, efavirenz, tenofovir disoproxil fumarate, or a combination comprising one or more of the foregoing inhibitors.
7. The pharmaceutical composition of Clause2, wherein the protease inhibitor comprises amprenavir, atazanavir, indinavir, nelfinavir, ritonavir, saquinavir, or saquinavir mesylate, or a combination comprising one or more of the foregoing protease inhibitors.
8. The pharmaceutical composition of any one of Clauses 1 to 7 prepared as an oral dosage form.
9. The pharmaceutical composition of Clause 8 prepared as a tablet or capsule.
10. An oral dosage form comprising Elvucitabine and a second active agent, wherein the second active agent is an immunomodulatory compound or an antiviral agent or a combination comprising one or more of the foregoing active agents, wherein the dosage form comprises from about 2.5 mg to not more than 6.5 mg Elvucitabine.
11. An oral dosage form comprising Elvucitabine and a second active agent, wherein the second active agent is an immunomodulatory compound or an antiviral agent or a combination comprising one or more of the foregoing active agents and wherein the dosage form provides an Elvucitabine C*ₘₐₓ* of not more than 45 micrograms/ L.
12. The oral dosage form of Clause 11 where the dosage form provides an Elvucitabine C*ₘₐₓ* of more than 2 micrograms/ L and less than 40 micrograms/ L.
13. The oral dosage form of Clause 10 comprising from about 2.5 to about 5 mg Elvucitabine and a second active agent, wherein the second active agent is a tyrosine kinase inhibitor, a CCR5 inhibitor, a non-nucleoside reverse transcriptase inhibitor, a nucleoside reverse transcripatase inhibitor, a protease inhibitor, a fusion inhibitor, an integrase inhibitor, a glucocorticoid, thalidomide, an interferon, IL-2, or a hematopoietin, or a combination of one or more of the foregoing active agents.
14. The oral dosage form of any one of Clauses 10 to 13, wherein the oral dosage is in the form of a tablet or capsule.
15. A packaged pharmaceutical composition comprising the dosage form of any one of Clauses 10 to 14 in a container and instructions for using the dosage fonn to treat a viral infection.
16. The packaged pharmaceutical composition of Clause 15, wherein the Elvucitabine and the second active agent are combined in a single dosage form.
17. The packaged pharmaceutical composition of Clause 15, wherein the Elvucitabine and the second active agent are in separate dosage forms.
18. The packaged pharmaceutical composition of Clause 15, wherein the instructions comprise instructions for administering about 2.5 mg to about 10 mg Elvucitabine per day.
19. The packaged pharmaceutical composition of Clause 15, wherein the instructions comprise instructions for administering about 10 mg to about 20 mg Elvucitabine per 48 hours.
20. The packaged pharmaceutical composition of Clause 15, wherein the instructions comprise instructions for administering about 40 mg to about 100 mg Elvucitabine per week.
21. A method of treating a viral infection in a patient, comprising administering Elvucitabine and a second active agent to the patient, wherein the second active agent is an immunomodulatory compound, an antiviral agent, or a combination comprising one or more of the foregoing active agents, with the proviso that the antiviral agent is not interferon, a nucleoside, or a nucleoside analog.
22. A method of treating a viral infection in a patient, comprising administering an effective amount of Elvucitabine and a second active agent to the patient, wherein the second active agent is an immunomodulatory agent, an antiviral agent, or a combination comprising one or more of the foregoing agents, wherein not more than 6.5 mg Elvucitabine are administered per day.
23. The method of Clause 21 or 22 wherein the antiviral agent is a tyrosine kinase inhibitor, a CCR5 inhibitor, a non-nucleoside reverse transcriptase inhibitor, a protease inhibitor, an integrase inhibitor, or a combination comprising one or more of the foregoing agents.
24. The method of Clause 23, wherein about 2.5 mg to about 10 mg of Elvucitabine is administered per day.
25. The method of Clause23, wherein about 5 mg to about 20 mg Elvucitabine is administered per 48 hour interval.
26. The method of Clause 23, wherein about 40 mg to about 100 mg of Elvucitabine is administered per week.
27. The method of Clause 21 or 22, wherein the Elvucitabine and the second active agent are both administered once per day.
28. The method of Clause 21 or 22, wherein the Elvucitabine is administered once every 2 to 7 days, and the second active agent is administered once per day.
29. The method of Clause 23, wherein the non-nucleoside reverse transcriptase inhibitor comprises efavirenz, tenofovir, or a combination comprising one or more of the foregoing inhibitors.
30. The method of Clause23, wherein the protease inhibitor comprises amprenavir, indinavir, saquinavir, nelfinavir, ritonavir, a blend of lopinavir and ritonavir, atazanavir, or a combination comprising one or more of the foregoing protease inhibitors.
31. The method of any one of Clauses 21 to 30, wherein the viral infection is an HBV or HIV infection.
32. The manufacture of a medicament for the treatment of a viral infection, comprising Elvucitabine and a second active agent viral infection, wherein the second active agent is an immunomodulatory compound, an antiviral agent, or a combination comprising one or more of the foregoing active agents, with the proviso that the antiviral agent is not interferon, a nucleoside, or a nucleoside analog.
33. The manufacture of a medicament for the treatment of a viral infection comprising Elvucitabine together with a second active agent, wherein the second active agent is an immunomodulatory agent, an antiviral agent or a combination comprising one or more of the foregoing agents, wherein a single dosage form of the medicament contains not more than 6.5 mg Elvucitabine.

## Claims

1. A pharmaceutical composition comprising elvucitabine, lactose monohydrate, and anhydrous lactose, in which the lactose monohydrate and anhydrous lactose taken together comprise about 60 percent to about 90 percent of the pharmaceutical composition by weight.

2. The pharmaceutical composition of Claim 1, wherein the composition comprises
(i) less than 2.0% magnesium stearate, or
(ii) less than 1.0% magnesium stearate, or
(iii) less that 0.5% magnesium stearate.

3. The pharmaceutical composition of Claim 1, wherein the composition comprises about 0.05 to about 0.5 percent magnesium stearate.

4. The pharmaceutical composition of Claim 3, wherein the composition is blended 23 - 28 minutes after adding the magnesium stearate.

5. The pharmaceutical composition of Claim 1 or 2 wherein the composition comprises 2.5 mg to about 10 mg Elvucitabine.

6. The pharmaceutical composition of Claim 3, wherein the composition is in tablet form and comprises 5.0 mg elvucitabine per tablet.

7. The pharmaceutical composition of Claim 1, wherein the elvucitabine
(i) has a mean particle size of less than 200 microns; or
(ii) wherein 90% of elvucitabine particles are between 50 and 150; or
(iii) wherein 98% of elvucitabine particles are between 25 and 180 microns.

8. The pharmaceutical composition of Claim 1, formulated for administration once per 48 hours and contain about 10 mg elvucitabine, or about 15 mg elvucitabine, or about 20 mg elvucitabine.

9. The pharmaceutical composition of Claim 1, formulated for administration every 2 to 7 days and containing 15 mg to 60 mg elvucitabine.

10. The pharmaceutical composition of any one of Claims 1 to 9, wherein the formulation is in the form of a tablet and the tablet is coated.

11. The pharmaceutical composition of Claim 10, where the formulation is coated with a seal coat, and enteric coat, and an overcoat.
